# EUROPEAN PATENT SPECIFICATION

(11) **EP 3 755 447 B1**
(45) Date of publication and mention of the grant of the patent: **06.03.2024**
(21) Application number: 19710843.4
(22) Date of filing: 21.02.2019
(51) Int. Cl.: B01D 15/16, G01N 30/86

(54) **DETERMINING CONDITIONS FOR PURIFICATION OF PROTEINS**
BESTIMMUNG VON BEDINGUNGEN FÜR DIE AUFREINIGUNG VON PROTEINEN
DÉTERMINATION DE CONDITIONS DE PURIFICATION DE PROTÉINES

(30) Priority: 21.02.2018 US 201862633584 P
(43) Date of publication of application: 30.12.2020
(73) Proprietor: Just-Evotec Biologics, Inc., Seattle, WA 98109 (US)
(72) Inventor: SHAVER, Jeremy, Martin, Seattle, WA 98109 (US); AMIMEUR, Tileli, Seattle, WA 98109 (US); GILLESPIE, Ron, Seattle, WA 98109 (US); KETCHEM, Randal, R., Seattle, WA 98109 (US); GARCIA, Fernando, Seattle, WA 98109 (US)
(74) Representative: Murgitroyd & Company
(86) International application number: PCT/US2019/019050
(87) International publication number: WO 2019/165148

(56) References cited:
- US-A1- 2002 010 566
- US-A1- 2004 034 477
- US-A1- 2006 096 924
- US-A1- 2008 066 530
- US-A1- 2016 367 911
- US-A1- 2017 322 190

## Description

### CROSS-REFERENCE TO RELATED APPLICATION(S)

This application is claims priority to U.S. Provisional Application No. 62/633,584 filed on February 21, 2018 and entitled "Determining Conditions for Purification of Proteins".

### BACKGROUND

Proteins are comprised of a sequence of amino acids that are linked via chemical bonds. The amino acid sequence of a particular protein is based on a sequence of nucleotides in the deoxyribonucleic acid (DNA) from which the protein is expressed. The functionality and structure of a protein can be based on the amino acid sequence of the protein. Proteins can have a variety of functions within an organism, such as regulation of enzymatic activity or cellular signaling. Some proteins can also be used therapeutically to treat a biological condition. For example, proteins, such as an antibody, can, in some cases, bind to a pathogen to target the pathogen for destruction by other agents in the organism, such as T cells or macrophages. In another example, proteins can bind to a molecule to transport the molecule to a targeted location in an organism to alleviate phenotypes of a biological condition.

Proteins can be expressed through a variety of processes, but the expressed proteins are often contained in a solution along with impurities that are associated with the expression of the proteins. Purification of the expressed proteins can take place utilizing a number of techniques. Optimization of the purification process for proteins can be a time consuming and costly undertaking because many rounds of experimentation may be needed utilizing different types of chromatographic techniques and conditions to identify the purification conditions that provide the highest yields and purity.

US 2002/010566 A1 discloses a method of optimizing conditions for the HPLC chromatographic purification of a solute, in particular predicting the solute peak width accounting for peak compression. The method disclosed is not explicitly designed for proteins or biomolecules, simply a 'solute'.

US 2016/367911 A1 discloses a method of optimizing conditions for the chromatographic purification of a target protein by generating a model created from data obtained by subjecting said target protein to a variety of chromatography conditions.

US 2017/322190 A1 discloses a method of optimizing conditions for the chromatographic purification of a target protein by generating a model created from data obtained by subjecting said target protein to a variety of chromatography conditions.

US 2006/096924 A1 discloses a method of optimizing conditions for the chromatographic purification of a target protein by generating a model created from data obtained by subjecting said target protein to a variety of chromatography conditions.

US 2008/066530 A1 discloses a method of optimizing conditions for the chromatographic purification of a target protein by generating a model created from data obtained by subjecting said target protein to a variety of chromatography conditions.

US 2004/034477 A1 discloses a method of generating optimal conditions for the chromatographic purification of a target compound by comparing the structure of the target compound with other compounds in a database and using similar conditions to the already optimized conditions known for compounds with a similar structure to the target compounds. It is apparent to those skilled in the art that this method is far more suitable for smaller organic compounds than larger and significantly more complex biomolecules or proteins.

### SUMMARY

The invention is a method and system as defined in the appended claims.

### BRIEF DESCRIPTION OF THE DRAWINGS

FIG. 1 is a diagram of some implementations of an architecture to determine conditions to optimize purification of proteins.
FIG. 2 illustrates some implementations of techniques to determine conditions to optimize purification of proteins using data obtained from a multi-well plate.
FIG. 3 illustrates some implementations of techniques to determine models to predict purity and yield of proteins under various purification conditions.
FIG. 4 illustrates some implementations of techniques to utilize a plurality of models to determine optimal conditions for purification of proteins.
FIG. 5 illustrates some implementations of a system to determine purification conditions for proteins using one or more chromatographic processes.
FIG. 6 is a flow diagram of an example process to determine purification conditions for proteins using one or more chromatographic processes.
FIG. 7 illustrates a number of user interfaces that indicate yield and purity for a protein with respect to a number of pH levels and salt concentrations.
FIG. 8 illustrates results from different models that can be used to determine yield and purity for proteins using multi-mode chromatography at various pH levels and salt concentrations.
FIG. 9 shows a 96-well filter plate configuration.
FIG. 10 shows a comparison of solution stability for 2 mAbs for the range of downstream process conditions evaluated.
FIG. 11 illustrates thermal stability by DSF for the range of downstream process conditions evaluated.
FIG. 12, panels A to D, and FIG. 13 illustrate an example of data generated during a single batch binding experiment.
FIG. 14 illustrates purity versus yield for paired modes across entire downstream process.

### DETAILED DESCRIPTION

The concepts described herein are directed to determining optimized conditions for the purification of proteins. In particular, the techniques and systems described herein are related to determining conditions for chromatographic processes that can be utilized to purify proteins after the proteins have been expressed. In some implementations, the systems can utilize machine learning techniques to determine the types of chromatographic processes and the conditions for the chromatographic processes that lead to optimal yields and purity for various proteins.

Column chromatography can utilize various principles to separate proteins from impurities in a solution. Column chromatography can include placing an amount of material into a vertically arranged column, where the material interacts with molecules that flow through the column. As different molecules interact with the material placed in the column, the molecules move through the column at different rates. Thus, some molecules can move more slowly through the column than others leading to a separation of the molecules moving through the column. The different molecules can then be captured as they exit the column. Molecules can be separated based on size, shape, charge, hydrophobicity, combinations thereof, or other characteristics that can cause interactions between the molecules and the material placed into the column. In various implementations, the material placed into the column that interacts with the molecules moving through the column can be referred to as a stationary phase material. The molecules moving through the column can be included in what can be referred to as a mobile phase or eluent that passes over the stationary phase. Some chromatographic processes can include ion exchange chromatography, affinity chromatography, high pressure liquid chromatography (HPLC), hydrophobic interaction chromatography (HIC), mixed mode chromatography (MMC), reverse phase chromatography, (RPC), size exclusion chromatography, and the like.

The cost and effectiveness of a protein purification process using column chromatography can depend on the type of chromatographic process utilized to purify the proteins because some chromatographic techniques can be more effective at purifying certain proteins than other chromatographic techniques. The type of chromatographic process utilized to purify a protein can also correspond to the use of certain stationary phase materials that can impact the cost of protein purification. In some instances, multiple chromatography processes can be utilized to purify proteins; however, each chromatographic step utilized in the protein purification process can result in some amount of lost protein.

Typically, selection of chromatographic processes and the determination of the conditions to purify proteins is based on knowledge available to those skilled in the art, such as journal articles, research papers, and textbooks, and a trial and error process is performed based on this knowledge whereby a particular protein is purified using one or more chromatographic processes under a variety of conditions. The use of the conventional process to select chromatographic techniques and conditions is limited in scope and inefficient. That is, for each protein to be purified, an amount of stationary phase material and an amount of the protein is utilized in order to determine a possible protocol for purification of the protein. Since an amount of stationary phase material and an amount of protein is utilized to identify chromatographic processes to purify a protein, the number of combinations of conditions, such as pH and salt concentration of the mobile phase solution, is also limited. In particular, the cost of the protein and stationary phase material, as well as the time utilized, for more than a few trial runs to determine the conditions that provide the best yield and purity for the protein can become prohibitive.

The techniques and systems described herein overcome the problems with the conventional techniques of determining protein purification conditions by collecting data with respect to different chromatography techniques under various conditions for a limited number of proteins and then utilizing machine learning to optimize the purification process for many other additional proteins. In some implementations, models can be developed to predict the optimal chromatographic conditions for a protein based on particular data obtained about the protein. For example, models to determine yield and purity for a number of chromatographic techniques under various conditions can be based on sequences of the proteins. Additionally, models to determine yield and purity for a number of chromatographic techniques under a number of conditions can be based on secondary structures associated with the proteins. In other situations, models to determine yield and purity for a number of chromatographic techniques with respect to certain conditions can be based on values of biophysical properties of the proteins that are measured using analytical techniques.

The models developed to determine the yield and purity of proteins can be evaluated together to determine a particular chromatographic technique or a particular combination of techniques that provide the optimal yield and purity for the protein. The cost of implementing the chromatographic techniques can also be evaluated when determining a particular chromatographic technique or combination of chromatographic techniques that provide the optimal yield and purity for the protein. The cost of a chromatographic technique can be based on a cost of the stationary phase material, a cost of the mobile phase, a size of the column utilized, how difficult the stationary phase material is to work with, yield of an individual chromatography step, combined yield of several chromatography steps, or combinations thereof.

Additionally, techniques and systems described herein can minimize the amount of data needed to generate the models utilized to predict the yield and purity for proteins. In various implementations, the chromatography conditions utilized in the columns can be determined from a minimum number of combinations of chromatography conditions that can be simulated using a multi-well plate. The minimum number of combinations of chromatography conditions can be identified by minimizing differences between reference data and a subset of wells of the multi-well plate under various chromatography conditions for different stationary phase materials. In particular, subsets of chromatography conditions for a stationary phase material for some proteins can be utilized across a wider range of proteins to provide yield and purity data to generate models that predict protein yield and purity for particular stationary phase materials.

By utilizing the techniques and systems described herein, the optimal conditions for purification of proteins can be determined for a number of proteins without having to perform actual runs of chromatographic processes for each of the proteins being evaluated under a number of different conditions. Thus, the cost of determining optimal conditions for purification of a protein is reduced and the efficiency is increased. Additionally, by minimizing the amount of data obtained to generate the models for determining the optimal conditions that maximize yield and purity for the purification of proteins, the amount of computing resources, such as processing resources and memory resources, is minimized.

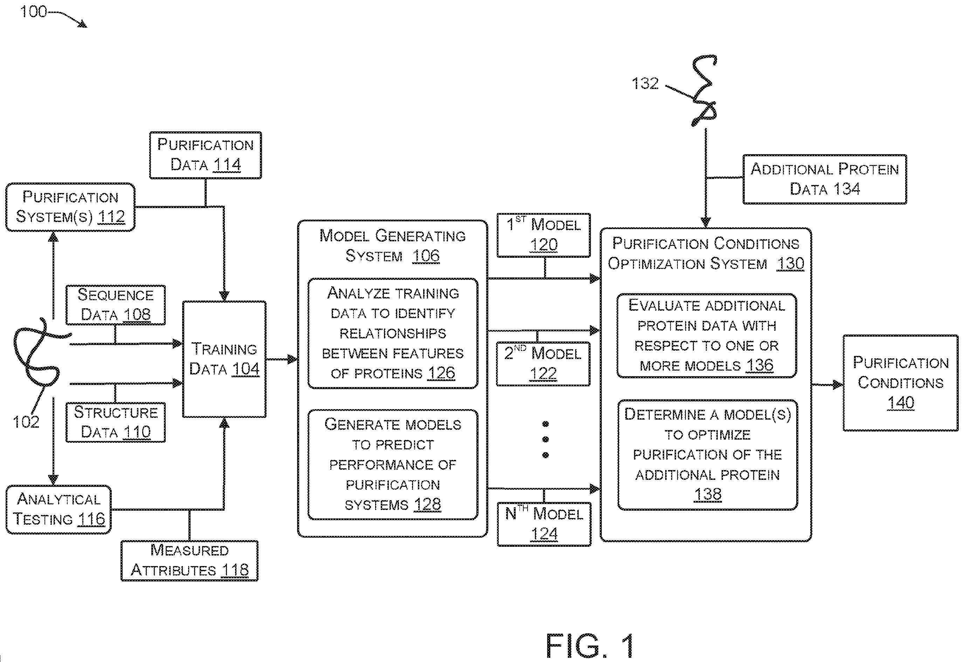

FIG. 1 is a diagram of some implementations of an architecture 100 to determine conditions to optimize purification of proteins. The architecture 100 can evaluate a number of proteins, such as representative protein 102, to produce training data 104. The training data 104 can be provided to a model generating system 106 to produce a number of models to predict measures of performance of a number of stationary phase materials for various chromatographic techniques. The measures of performance can include at least one of yield or purity for an individual stationary phase material. In some implementations, the protein 102 can include an antibody. In an illustrative example, the protein 102 can include an IgG antibody.

The training data 104 can include sequence data 108 that indicates at least a portion of an amino acid sequence of the protein 102. The sequence data 108 can indicate the amino acid at individual positions of the sequence of the protein 102. In various implementations, the sequence data 108 can be determined by mass spectrometry. Particular techniques for determining a protein sequence using mass spectrometry can be found in Hunt, D F et al. "Protein Sequencing by Tandem Mass Spectrometry." Proceedings of the National Academy of Sciences of the United States of America 83.17 (1986): 6233-6237. Print. Additionally, the sequence data 108 can be determined using Edman degradation as described in Berg JM, Tymoczko JL, Stryer L. Biochemistry. 5th edition. New York: W H Freeman; 2002. Section 4.2, Amino Acid Sequences Can Be Determined by Automated Edman Degradation. Available from: https://www.ncbi.nlm.nih.gov/books/NBK22571/. Further, the sequence data 108 can be determined from a sequence of nucleotides associated with the protein 102, such as a deoxyribonucleic acid (DNA) sequence or a ribonucleic acid (RNA) sequence associated with the protein 102, as described in Smith, A. (2008) Nucleic acids to amino acids: DNA specifies protein. Nature Education 1(1): 126.

The training data 104 can also include structure data 110 that indicates structural features of the protein 102. The structure data 110 can indicate secondary features of the protein 102, tertiary features of the protein 102, or both. The structure data 110 can indicate features of the three-dimensional structure of the protein 102, such as turns and/or loops. In certain implementations, the structure data 110 can indicate α-helixes, β-turns, β-sheets, Ω-loops, or combinations thereof. The structure data 110 can also indicate hydrophobic regions of the protein 102, polar regions of the protein 102, regions of the protein 102 having certain charges, or combinations thereof. The structure data 110 can indicate a number of positions associated with a type of secondary structure or a type of tertiary structure of the protein 102. Secondary structure of the protein 102 can be determined by a number of methods including those described in Determination of the secondary structure of proteins by laser Raman spectroscopy; J. L. Lippert, D. Tyminski, and P. J. Desmeules; Journal of the American Chemical Society 1976 98 (22), 7075-7080 DOI: 10.1021/ja00438a057.

The training data 104 can also include data obtained from analyzing the protein 102 by one or more purification systems 112 to produce purification data 114. The one or more purification systems 112 can include one or more chromatographic techniques. In particular implementations, the protein 102 can be expressed and then purified utilizing at least one chromatographic technique under various conditions, such as various pH levels and salt concentrations. The purification data 114 can also include yield and purity data obtained from utilizing the one or more purification systems 112 with respect to the protein 102. The one or more purification systems 112 can include chromatographic processes, such as ion exchange chromatography (IEX), affinity chromatography, hydrophobic interaction chromatography (HIC), multi-mode chromatography (MMC), reversed phase chromatography (RPC), size exclusion chromatography (SEC), or combinations thereof. The use of other chromatographic techniques is also contemplated in other implementations. In some implementations, the purification data 114 can be determined through analyzing purity and yield values obtained from multi-well plates with respect to various stationary phase materials. The stationary phase materials analyzed with regard to the purification systems 112 can include polymeric materials, such as resins. In particular implementations, the stationary phase materials analyzed with regard to the purifications systems 112 can include silica-containing materials. In some illustrative examples, the stationary phase materials utilized with regard to the purifications systems 112 can include silica, crosslinked agarose, methacrylate, hydrophilic polyvinyl ether, cellulose, hydrogel, polymethacrylate, hydroxyapitite, or combinations thereof.

The training data 104 can also include data obtained by subjecting the protein 102 to analytical testing 116 to produce measured attributes 118. The analytical testing can include a number of assays that produce data about the protein 102. The measured attributes 118 can include a molecular weight of the protein 102 that can be determined using size exclusion chromatography. The measured attributes 118 can also include turbidity measured using a UV-Vis spectrophotometer. Additionally, the measured attributes 118 can include a measure of stability of the protein 102 that is determined by differential scanning fluorimetry (DSF). The stability of the protein 102 can also be measured using a Chemical Unfolding assay or a Viscosity assay. Further, the measured attributes 118 can include measures of interactions between regions of the protein 102 as determined by self-interaction nanoparticle spectroscopy (SINS).

The training data 104 can be provided to a model generating system 106 that can determine models to predict measures of performance for various protein purification techniques. Individual models produced by the model generating system 106 can be associated with a respective stationary phase material. For example, a first model 120 can predict yield and/or purity for a first stationary phase material. In addition, a second model 122 can predict yield and/or purity for a second stationary phase material. The model generating system 106 can generate up to an N^{th} model 124 that can predict yield and/or purity for an N^{th} stationary phase material. In some implementations, the first stationary phase material can be associated with a first chromatographic technique and the second stationary phase material can be associated with a second chromatographic technique that is different from the first chromatographic technique. In other implementations, the first stationary phase material can be different from the second stationary phase material, but associated with a same chromatographic technique. In illustrative examples, the first model 120 can be produced with respect to a stationary phase material associated with an anion exchange chromatographic technique, the second model 122 can be produced with respect to a stationary phase material associated with a hydrophobic interaction chromatographic technique, and the N^{th} model 124 can be produced with respect to a stationary phase material associated with a mixed-mode chromatographic technique.

At 126, the model generating system 106 can analyze the training data 104 to identify relationships between features of a group of proteins that includes the representative protein 102. In some implementations, the model generating system 106 can determine relationships between sequences of the proteins and one of more of the measured attributes. In various implementations, the model generating system 106 can determine relationships between yield and/or purity of the one or more purification systems 112 and the sequence data 108, the structure data 110, and/or the measured attributes 118 for a group of proteins that includes the protein 102. For example, the model generating system 106 can determine that proteins having a particular sequence at a range of positions can have values for turbidity within a specified range. In another example, the model generating system 106 can determine that proteins having a range of molecular weights are correlated with relatively higher yields when purified using MMC in relation to yields achieved when purified using anion exchange chromatographic techniques. The model generating system 106 can also determine relationships between purification conditions, such as pH levels and/or salt concentrations, and the sequences of the proteins used to produce the training data 104, structures of the proteins used to produce the training data 104, measured attributes of the proteins used to produce the training data 104, or combinations thereof. Further, the model generating system 106 can determine relationships between purification conditions and the types of stationary phase materials utilized in the one or more purification systems 112.

At 128, the model generating system 106 can generate a number of models to predict measures of performance, such as yield and/or purity, of the one or more purification systems 112. In particular, the models produced by the model generating system 106, such as the models 120, 122, 124, can predict measures of performance for proteins that are different from the proteins used to produce the training data 104. That is, the models 120, 122, 124 can predict measures of performance for proteins that have different sequences than the proteins used to produce the training data 104. The models generated by the model generating system 106 can include variables associated with one or more of the sequence data 108, the structure data 110, purification conditions, or one or more of the measured attributes 118. The models can also include coefficients for the variables that indicate relative weights of the variables with respect to one another. The variables and coefficients included in the models can be based on the relationships between the various features of the training data that are determined at operation 126. In an illustrative example, the models 120, 122, 124 can include polynomial models.

The model generating system 106 can perform an iterative process to determine the models 120, 122, 124. For example, the model generating system 106 can determine a polynomial function for a particular stationary phase material and test the polynomial function against the training data 104. If the polynomial function does not produce measures of performance consistent with the training data 104, the model generating system 106 can modify the variables and/or coefficients of the initial polynomial function to produce an additional polynomial function. The model generating system 106 can then evaluate the additional polynomial function with respect to the training data 104. The model generating system 106 can evaluate a number of iterations of a model for each stationary phase material until the results produced by the models correspond to the training data 104 within a threshold amount. That is, the model generating system 106 can evaluate models for stationary phase materials to minimize error between the predicted results and actual results. In an illustrative example, the model generating system 106 can iteratively test models for individual stationary phase materials until a local minimum is achieved. In additional illustrative examples, the model generating system 106 can iteratively test models for individual stationary phase materials until a global minimum is achieved. Further, the model generating system 106 can iteratively test models for individual stationary phase materials until a specified number of training cycles have been completed. In situations where an accuracy of an individual stationary phase material is less than a threshold accuracy, additional training cycles can be performed and in some situations parameters of the model can be tuned during the additional training cycles. In particular implementations, hyperparameters of the model can be tuned for additional training cycles in scenarios where an accuracy of the model for an individual stationary phase material is less than a threshold accuracy.

The models generated by the model generating system 106 can be provided to a purification conditions optimization system 130. In the illustrative example of FIG. 1, the purification conditions optimization system 130 can utilize the models 120, 122, 124, or combinations thereof, to optimize the purification conditions for proteins that are different from the proteins utilized to produce the training data 104. In an illustrative example, the purification conditions optimization system 130 can determine optimizing conditions for purification of an additional protein 132. The purification conditions optimization system 130 can obtain additional protein data 134 that corresponds to the additional protein 132. The additional protein data 134 can include a sequence of the additional protein 132, one or more structures of the additional protein 132, measured attributes of the additional protein 132, or combinations thereof. In some implementations, the additional protein 132 can have some similarities with respect to the protein 102. For example, both the protein 102 and the additional protein 132 can be antibodies. In another example, both the protein 102 and the additional protein 132 can be IgG antibodies. In other examples, a molecular weight of the additional protein 132 can be within at least about 2% of the molecular weight of the protein 102, at least about 5% of the molecular weight of the protein 102, at least about 10% of the molecular weight of the protein 102, at least about 15% of the molecular weight of the protein 102, at least about 20% of the molecular weight of the protein 102, or at least about 25% of the molecular weight of the protein 102.

At 136, the purification conditions system 130 can evaluate the additional protein data 134 with respect to one or more of the models generated by the model generating system 106. In particular implementations, the purification conditions optimization system 130 can determine yield and purity values for the additional protein 132 with respect to a number of stationary phase materials based on the additional protein data 134. For example, the purification conditions optimization system 130 can evaluate the additional protein data 134 with respect to the first model 120, the second model 122, and the N^{th} model 124 to determine yield and purity values for the additional protein 132 for each respective stationary phase material associated with the models 120, 122, 124.

At 138, the purification conditions optimization system 130 can determine one or more models to optimize purification of the additional protein 132. In particular implementations, the purification conditions optimization system 130 can produce purification conditions 140 for the additional protein 132. The purification conditions 140 can indicate one or more chromatographic techniques to purify the additional protein 132. The purification conditions 140 can also indicate one or more pH values and one or more salt concentrations at which to perform the chromatographic techniques identified by the purification conditions optimization system 130.

The purification conditions optimization system 130 can compare the yield and purity values produced for the additional protein 132 by one or more of the models generated by the model generating system 106 to determine a model that maximizes values for yield and purity. In some implementations, the purification conditions optimization system 130 can determine a combination of models that maximizes values for the purity and yield of the additional protein 132. In an illustrative example, the purification conditions optimization system 130 can determine that a stationary phase material associated with the first model 120 can maximize yield and purity values for the additional protein. In another illustrative example, the purification conditions optimization system 130 can determine that purifying the additional protein 132 utilizing a first stationary phase material associated with the first model 120 followed by purifying the additional protein 132 with a second stationary phase material associated with the second model 122 can maximize yield and purity values for the additional protein 132. Additionally, the purification conditions and optimization system 130 can determine the purification conditions 140 based at least partly on cost of a number of stationary phase materials. To illustrate, the purification conditions optimization system 130 can determine the purification conditions 140 based at least partly on yield values, purity values, and cost of the stationary phase materials associated with the models generated by the model generating system 106, such as models 120, 122, 124.

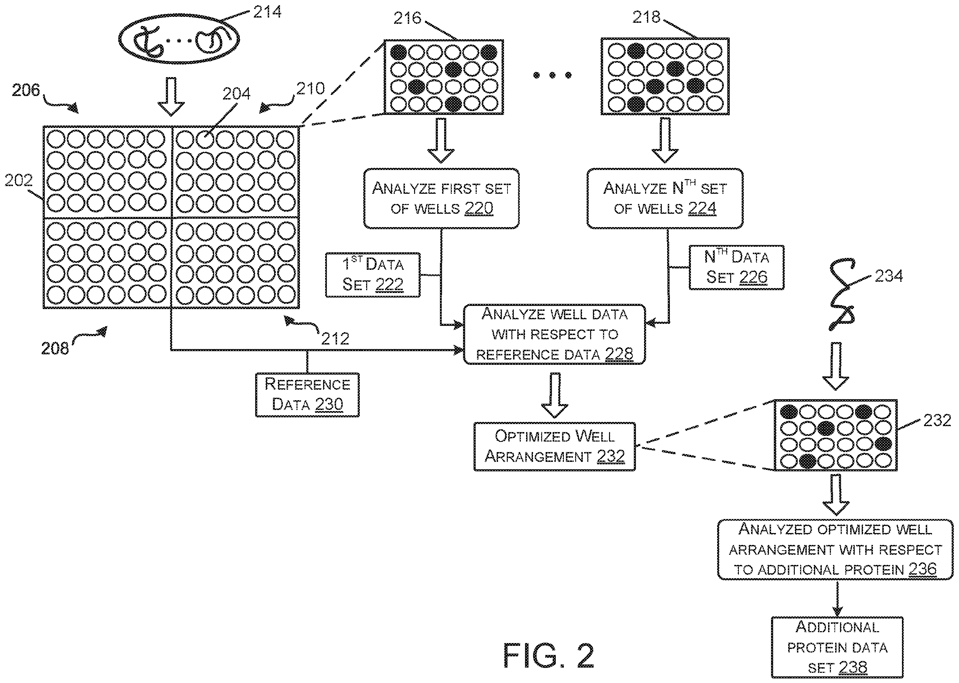

FIG. 2 illustrates some implementations of techniques to determine conditions to optimize purification of proteins using data obtained from a multi-well plate 202. In the illustrative example of FIG. 2, the wells of the multi-well plate 202, such as representative well 204, can be divided into quadrants. For example, the multi-well plate 202 can include a first quadrant 206, a second quadrant, 208, a third quadrant 210, and a fourth quadrant 212. In the illustrative example of FIG. 2, each quadrant can be associated with a different stationary phase material. In other implementations, the multi-well plate 202 can be divided into a different number of portions, such as two portions, three portions, or six portions, to accommodate a different number of stationary phase materials. Additionally, although each quadrant in the multi-well plate 202 includes a 4 x 6 matrix corresponding to four different salt concentrations and six different pH levels, the matrices associated with each portion of the multi-well plate 202 can be different. To illustrate, the 4 x 6 matrices of the multi-well plate 202 can correspond to four different pH levels and six different salt concentrations. In an additional example, a multi-well plate can be divided into three portions that each have an 8 x 4 matrix corresponding to eight pH levels and four salt concentrations or eight salt concentrations and four pH levels. Furthermore, although the illustrative example of FIG. 2 includes a multi-well plate 202 with 96 wells, other implementations can have multi-well plates with different numbers of wells.

The multi-well plate 202 can be utilized to provide purity and yield data with respect to a set of proteins 214 for a number of stationary phase materials. To illustrate, individual wells of the multi-well plate 202 can include a solution including an amount of a protein selected from the set of proteins 214 and an amount of a stationary phase material. The solution included in individual wells of the multi-well plate can also have a pH value and a salt concentration. The pH value can be based on an amount of acid included in the solution or an amount of base included in the solution. The salt concentration can be based on an amount of a salt included in the solution, such as an amount of NaCl included in the solution. Other salts can also be utilized in the solution, such as weak acid salts (e.g., CH₃COONa) or weak base salts (e.g., NHaCl). In particular implementations, the pH values of the solutions included in the wells of the multi-well plate can be from about 2.5 to about 8.5, from about 3 to about 8, from about 3 to about 5, or from about 5 to about 8. Additionally, the salt concentrations of the solutions included in individual wells of the multi-well plate 202 can be from about 0 millimolar (mM) to about 750 mM, from about 20 mM to about 650 mM, from about 25 mM to about 400 mM, from about 30 mM to about 200 mM, or from about 5 mM to about 100 mM.

Yield and/or purity determinations with respect to the stationary phase materials, pH values, and salt concentrations of the individual wells of the multi-well plate can be based at least partly on analyses of the solutions of the individual wells after a period of time. In particular implementations, colloidal stability of the solutions can be determined based on light scattering properties of the solution, such as turbidity, and chemical stability of the solution can be determined using a Chemical Unfolding assay. *See* Guillermo Senisterra, Irene Chau, and Masoud Vedadi. ASSAY and Drug Development Technologies. Volume 10, Issue 2, Apr 2012. Thermal stability of the solutions can also be determined, such as via differential scanning fluorimetry. Purity can be determined using size exclusion chromatography. Binding of the proteins of the set of proteins 214 to the stationary phase materials included in the individual wells of the multi-well plate 202 can also be utilized to determine yield and/or purity values for individual proteins of the set of proteins 212 under the conditions of the individual wells. In some cases, the solutions included in the individual wells can be run through a particular chromatographic process. Additionally, to determine an amount of protein bound to the stationary phase material included in individual wells of the multi-well plate 202, the amount of protein in the solutions of the individual wells can be compared with the initial amount of protein in the solution when the solution was first added to the individual well. Values of particular characteristics of the individual proteins included in the set of proteins 214 can also be analyzed after the solutions have been in the individual wells for a period of time to determine stability of the individual proteins, yield for the individual proteins, and/or purity for the individual proteins. To illustrate, in situations where the set of proteins 214 include antibodies, the high molecular weight (HMW) component can be analyzed to determine stability of individual proteins at various pH values and salt concentrations.

In certain implementations, each quadrant 206, 208, 210, 212 of the multi-well plate 202 can represent various experimental conditions for one or more proteins included in the set of proteins 214. For example, individual wells included in a particular quadrant 206, 208, 210, 212 can represent different combinations of salt concentrations and pH values for a particular stationary phase material. In other situations, multiple stationary phase materials can be evaluated at various pH values and salt concentrations in a particular quadrant 206, 208, 210, 212.

In implementations described herein, a subset of wells for one or more of the quadrants 206, 208, 210, 212 can be determined that represent the yield, purity, and/or stability of the protein for the aggregated wells of the quadrant. To illustrate, the techniques described with respect to FIG. 2 can identify five wells or six wells of the third quadrant 210 that represent the data that can be obtained from conditions associated with the twenty-four wells of the quadrant 210. By reducing the number of wells analyzed to determine data that can be utilized to predict yield, purity, and/or stability of individual proteins, the amount of protein and the amount of stationary phase material used is minimized. In the illustrative example of FIG. 2, a first set of wells 216 up to an N^{th} set of wells 218 can be analyzed to identify a minimum number of wells to determine yield, purity, and/or stability data for individual proteins of the set of proteins 214. The individual wells included in the first set of wells 216 up to the Nth set of wells 218 can be shown as having a gray color in the illustrative example of FIG. 2.

At 220, the first set of wells 216 can be analyzed to produce the first data set 222. The analysis of the first set of wells 216 can include determining yield and/or purity of the protein included in the individual wells of the first set of wells 216 under the pH and salt concentration values associated with the individual wells of the first set of wells 216 to produce the first data set 222. The analysis of the first set of wells 216 can also include determining certain characteristics of a protein included in the individual wells of the first set of wells after remaining in the respective wells for a period of time, such as a high molecular weight (HMW) component, to produce the first data set 222. Additionally, characteristics of the solution included in the individual wells of the first set of wells 216, such as a turbidity of the solution, can be analyzed to produce the first data set 222.

A number of additional sets of wells including different combinations of conditions can be analyzed until an N^{th} set of wells is analyzed at 224 to produce an N^{th} data set 226. In some cases, the combinations of wells that are analyzed for each round of analysis can include a same number of wells, but have different combinations of conditions. In particular implementations, the wells of the third quadrant 210 can be selected for evaluation according to a random or pseudo-random algorithm. In an illustrative example, a Monte Carlo algorithm can be utilized to determine the combinations of individual wells to evaluate for each round of analysis.

Individual data sets produced for each combination of wells can be analyzed, at 228, with respect to a reference data set 230. The reference data set 230 can be produced from a greater number of wells of the third quadrant than the number of wells included in the first set of wells 216 through the N^{th} set of wells 218. For example, the reference data set 230 can be produced from at least about 75% of the wells of the third quadrant 210, at least about 80% of the wells of the third quadrant 210, at least about 85% of the wells of the third quadrant 210, at least about 90% of the wells of the third quadrant 210, or at least about 95% of the wells of the third quadrant 210. In various implementations, the reference data set 230 can be derived from a set of wells included in the third quadrant that includes at least a portion of the wells included in each of the first set of wells 216 through the N^{th} set of wells 218. The reference data 230 can include data that corresponds with the data included in the first data set 222 through the N^{th} data set 226. To illustrate, the reference data set 230 can include yield and/or purity values for a number of the wells included in the third quadrant 210, data regarding the solution included in the wells of the third quadrant 210, and data indicating characteristics of the protein included in the wells of the third quadrant 210.

In particular implementations, at least a portion of the first data set 222 can be compared with at least a portion of the reference data set 230 and an amount of error between the at least a portion of the first data set 222 and the at least a portion of the reference data set 230 can be determined. Another group of wells can then be selected and at least a portion of data derived from the second group of wells can be compared with the at least a portion of the reference data set 230 and an amount of error between the data derived from the second group of wells and the reference data 230 can be determined. A number of additional groups of wells can be analyzed and the data derived from the additional groups of wells can be compared against the reference data 230. Each successive group of wells is analyzed in such a way to reduce the error between a newly selected group of wells and the reference data until a minimum error is attained. The combination of wells that is associated with the minimum error between the data generated from the combination of wells and the reference data is represented by the optimized well arrangement 232.

The optimized well arrangement 232 can be utilized to generate data for additional proteins that are different from the set of proteins 214 that were used to produce the optimized well arrangement 232. In the illustrative example of FIG. 2, an additional protein 234 can be placed into wells associated with the conditions corresponding to the wells of the optimized well arrangement 232. In particular, an amount of the additional protein 234 can be placed into wells of a multi-well plate at pH values, salt concentrations, and with an amount of stationary phase material that corresponds to the conditions of the optimized well arrangement 232. At 236, the additional protein can be analyzed with respect to the conditions of the optimized well arrangement 232 to produce an additional protein data set 238. The additional protein data set 238 can include yield and/or purity values for the wells of the optimized well arrangement 232 with respect to the additional protein 234. The additional protein data set 238 can also include data indicating characteristics of the solution included in the individual wells of the optimized well arrangement for the additional protein 234.

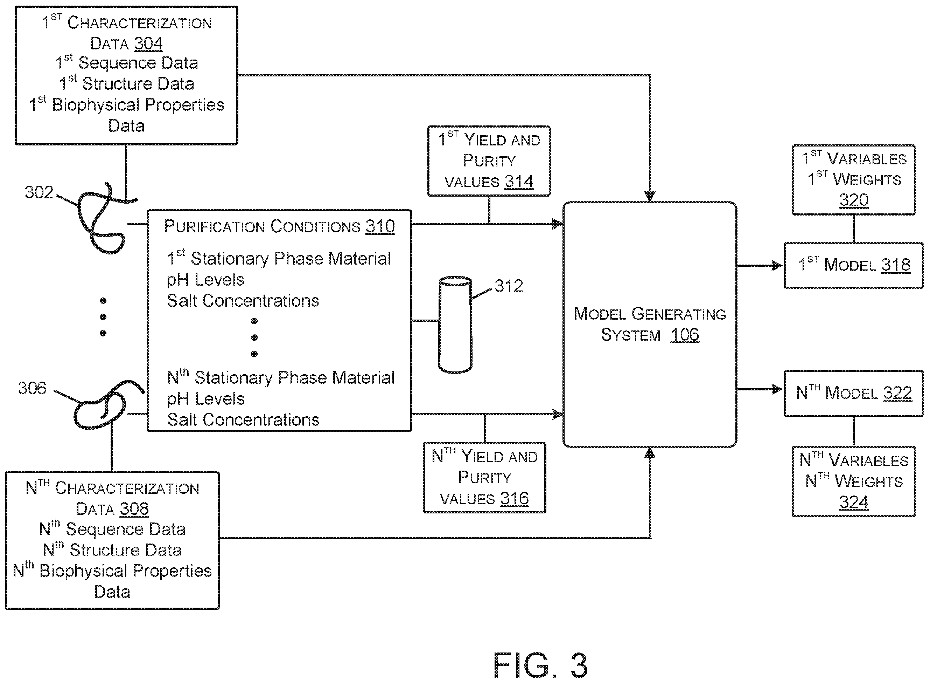

FIG. 3 illustrates some implementations of techniques to determine models to predict purity and yield of proteins under various purification conditions. In particular, a first protein 302 can be associated with first characterization data 304. The first characterization data 304 can include a sequence of the first protein 302, one or more secondary structures of the first protein 302, one or more tertiary structures of the first protein 302, or combinations thereof. The first characterization data 304 can also include values for biophysical properties of the first protein 302. The model generating system 106 can obtain the first characterization data 304 and utilize the first characterization data 304 to generate models to predict yield and purity for various proteins. The model generating system 106 can also obtain data regarding other proteins, up to an N^{th} protein 306, to generate the models utilized to predict yield and purity. In the illustrative example of FIG. 3, the model generating system 106 can also obtain N^{th} characterization data 308 that includes sequence data for the N^{th} protein 306, data for one or more secondary structures of the N^{th} protein, data for one or more tertiary structures of the N^{th} protein 306, biophysical properties data for the N^{th} protein 306, or combinations thereof.

In addition to protein characterization data, the model generating system 106 can utilize yield and purity data for the first protein 302 up to the N^{th} protein 306 to generate models to determine yield and purity values for additional proteins. For example, purification conditions 310 can be applied to a chromatography column 312 with respect to the first protein 302 up to the N^{th} protein 306. In this way, first purity and yield values 314 for the first protein 302 up to N^{th} purity and yield values for the Nth protein 306 can be collected with respect to multiple stationary phase materials at various combinations of pH levels and salt concentrations.

The model generating system 106 can analyze the first characterization data 304 up to the N^{th} characterization data 306 and the first yield and purity values 314 up to the N^{th} yield and purity values 316 to determine relationships between different variables included in the characterization data 304 ... 306 and the yield and purity values 314 ... 316. For example, the model generating system 106 can identify relationships between yield and purity values for proteins having particular sequence features under certain purification conditions, such as a particular stationary phase material, a range of pH values, and a range of salt concentrations. The model generating system 106 can also identify relationships between yield and purity values for proteins having values for one or more particular biophysical properties under certain purification conditions. Additionally, the model generating system 106 can identify relationships between yield and purity values for proteins having particular secondary structures at various positions under certain purification conditions.

The model generating system 106 can generate a first model 318 having first variables and first weights 320 up to an N^{th} model 322 having N^{th} variables and N^{th} weights 324. Individual models from the first model 318 up to the N^{th} model 322 can correspond to individual stationary phase materials that can be utilized to purify proteins in a chromatography column, such as the column 312. For example, the first model 318 can predict yield and purity values for a stationary phase material utilized in anion exchange chromatography. In another example, the N^{th} model 322 can predict yield and purity values for a stationary phase material utilized in multi-mode chromatography. The variables of the first model 318 up to the N^{th} model 322 can correspond to features of the characterization data for proteins. Additionally, the variables of the first model 318 can correspond to various pH levels and salt concentrations. To illustrate, the first model 318 can include variables related to protein sequence and the presence of various secondary structures with respect to a particular stationary phase material. The first model 318 can also include variables related to pH levels and salt concentrations with respect to the particular stationary phase material. The weights associated with the first model 318 up to the N^{th} model 322 can indicate an amount of impact of a variable on the yield and purity values.

In illustrative implementations, characterization data for additional proteins can be evaluated with respect to one or more of the models generated by the model generating system 106. In a particular example, a model generated by the model generating system 106 can be associated with a stationary phase material and have variables related to a number of hydrophobic amino acids included in a protein sequence, a number of beta sheets, differential scanning fluorimetry values, pH levels, and salt concentrations. Continuing with this example, characterization data for an additional protein that indicates a number of hydrophobic amino acids included in the sequence of the additional protein, a number of beta sheets included in the additional protein, and differential scanning fluorimetry values of the additional protein can be evaluated in conjunction with ranges of pH levels and salt concentrations to predict yield and purity values for the different stationary phase materials according to one or more of the models generated by the model generating system 106.

In various implementations, the model generating system 106 can generate models using principle component analysis (PCA) techniques. For example, the model generating system 106 can characterize the yield and purity values, such as the first yield and purity values 314 up to the Nth yield and purity values 316 as simply one PCA variable or as two PCA variables. The model generating system 106 can also determine respective weights for the variables. In this way, the first variables and first weights 320 included in the first model 318 and the Nth variables and Nth weights 324 included in the Nth model 322 can include one or more PCA variables and their respective weights. By implementing PCA techniques to determine variables and weights to include in the models, the model generating system 106 can reduce the computation power, such as processor cycles, utilized to predict yield and/or purity using the models.

In particular implementations, the model generating system 106 can generate models that can be implemented with a group of molecules having a set of characteristics based on the characterization data of the molecules and the chromatography technique used to purify the molecules. For example, molecules having particular sequences with variants at certain positions, molecules having a specified range of biophysical properties, and/or molecules having specified ranges of biophysical properties. In certain implementations, the model generating system 106 can generate separate models for subsets of the molecules included in a group of molecules. In an illustrative example, the model generating system 106 can generate a single model for a group of proteins having a specified set of characteristics that is purified using size exclusion chromatography. In another illustrative example, the model generating system 106 can generate multiple models for the same group of proteins that is purified using hydrophobic interaction chromatography. Specifically, the model generating system 106 can generate a first model to predict yield and/or purity of molecules included in the group of molecules that have a relatively high amount of retention with respect to the stationary phase material included in the chromatography and a second model to predict yield and/or purity of the molecules included in the group that have a relatively lower amount of retention with respect to the stationary phase material included in the chromatography column. The model generating system 106 can utilize a threshold amount of retention to determine the molecules included in a first group having a relatively high amount of retention in relation to the molecules included in a second group having a relatively low amount of retention. In some particular illustrative examples, retention of molecules with respect to a stationary phase material can be measured according to a Retention Factor, k. The retention of molecules with respect to a stationary phase material can also be measured according to time factors and/or volume factors, such as a retention time for a given volume of mobile phase material.

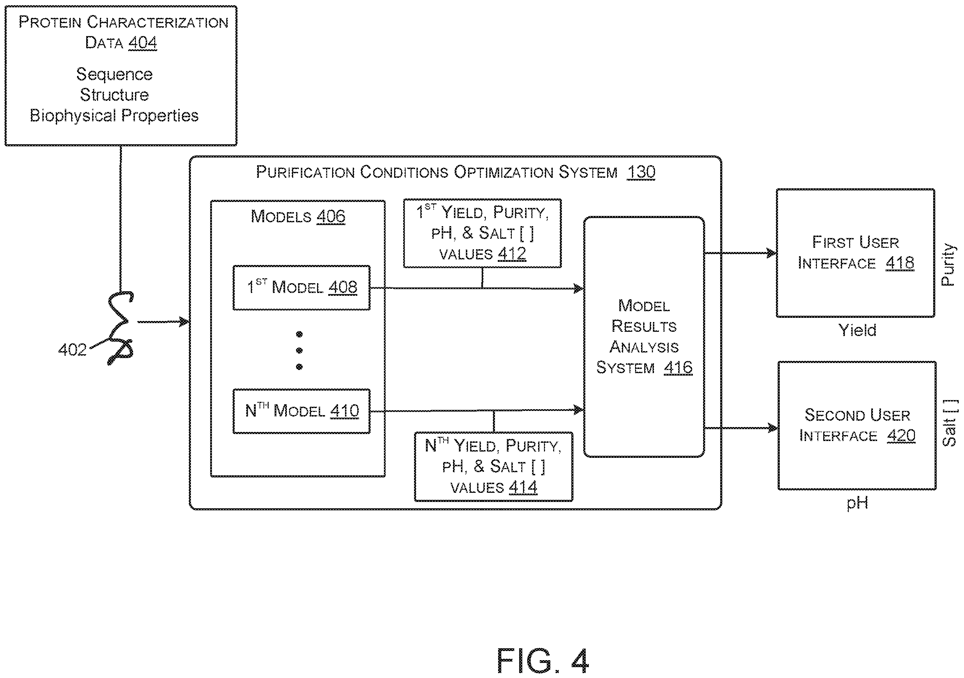

FIG. 4 illustrates some implementations of techniques to utilize a plurality of models to determine optimal conditions for purification of proteins. The models can be generated by the model generating system 106 of FIG. 1 and FIG. 3 and utilized by the purification conditions optimization system 130 to determine optimal conditions for the purification of various proteins. In the illustrative example of FIG. 4, optimal conditions for the purification of the protein 402 can be determined by the purification conditions optimization system 130. To illustrate, at least a portion of protein characterization data 404 that is associated with the protein 402 can be provided to the purification conditions optimization system 130. The portions of the protein characterization data 404 obtained by the purification conditions optimization system 130 can correspond to the data associated with the variables included in the various models 406. For example, in situations where one or more of the models 406 include a variable related to a number of polar amino acids included in a protein sequence, the protein characterization data 404 can include the number of polar amino acids included in the sequence of the protein 402. In some illustrative examples, chemical unfolding can be a factor included in the characterization data 404 that is predictive of purity and/or yield values. In other illustrative examples, hydrophobic regions can be predictive of purity and/or yield values. In still other illustrative examples, both chemical unfolding and hydrophobicity can be predictive of purity and/or yield values.

By analyzing at least portions of the protein characterization data 404 with respect to the models 406, such as a first model 408 up to an N^{th} model 410, the purification conditions optimization system 130 can produce yield and purity values for the protein 402 with respect to various pH levels and salt concentrations. In particular examples, the protein characterization data 404 can be evaluated with respect to the models 406 to determine respective yield and protein values for individual combinations of pH levels and salt concentrations. To illustrate, the purification conditions optimization system 130 can produce first yield, purity, pH, and salt concentration values 412 with respect to the first model 408 up to N^{th} yield, purity, pH, and salt concentration values 414 with respect to the N^{th} model 410. In an illustrative example, the purification conditions optimization system 130 can determine a yield of 90% and a purity of 85% for the protein 402 with respect to the first model 408 at a pH of 5.5 and a salt concentration of 300 mM.

The model results analysis system 416 can obtain the results produced by the models 406 and determine optimal conditions for the purification of proteins. In some cases, the optimal conditions for the purification of proteins can include a single stationary phase material at a particular pH level and a particular salt concentration. In other situations, the optimal conditions for the purification of proteins can include a combination of stationary phase materials at one or more pH levels and one or more salt concentrations. In particular scenarios, the optimal conditions for purification of the protein 402 can include purifying the protein 402 using a chromatographic technique having a first stationary phase material at a first pH level and a first salt concentration followed by using an additional chromatographic technique having a second stationary phase material at a second pH level and a second salt concentration. The model results analysis system 416 can also utilize cost data for the various stationary phase materials to determine the optimal conditions for purification of proteins. In various implementations, the model results analysis system 416 can utilize data regarding a size of a chromatography column and/or throughput data for a chromatography column to determine the optimal conditions for purification of proteins.

The model results analysis system 416 can produce one or more user interfaces that indicate yield and purity data for a number of combinations of pH levels and salt concentrations for different stationary phase materials. For example, the model results analysis system 416 can generate a first user interface 416 that has yield values on the x-axis and purity values on the y-axis. Each yield, purity pair included in the first user interface 416 can be associated with a particular stationary phase material at a specified pH level and salt concentration. Additionally, the model results analysis system 416 can generate a second user interface 420 that has pH values on the x-axis and salt concentration values on the y-axis. Each pH and salt concentration pair included in the second user interface 420 can be associated with particular yield and purity values.

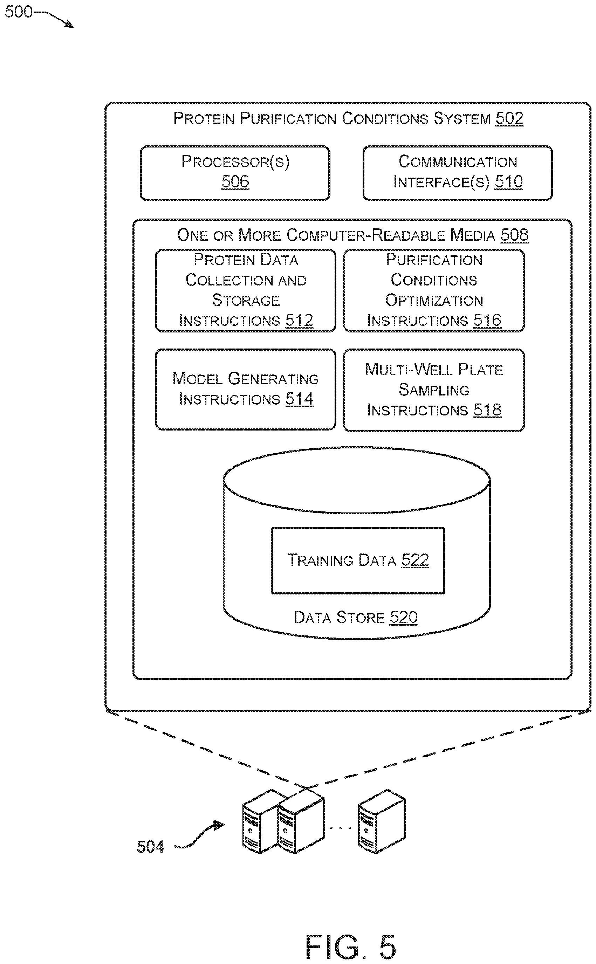

FIG. 5 illustrates some implementations of a system to determine purification conditions for proteins using one or more chromatographic processes. The system 500 includes a protein purification conditions system 502 that can be implemented by the one or more computing devices 504. In some implementations, the one or more computing devices 504 can be included in a cloud computing architecture that operates the one or more computing devices 504 on behalf of an entity implementing the protein purification conditions system 502, such as a user of the protein purification conditions system 502. In these scenarios, the cloud computing architecture can instantiate one or more virtual machine instances on behalf of the entity implementing the protein purification conditions system 502 using the one or more computing devices 504. The cloud computing architecture can be located remote from the entity implementing the protein purification conditions system 502. In additional implementations, the one or more computing devices 504 can be under the direct control of the entity implementing the protein purification conditions system 502. For example, the entity implementing the protein purification conditions system 502 can maintain the one or more computing devices 504 to perform operations related to generating one or more models and analyzing protein data with respect to the models to determine conditions to purify proteins. In various implementations, the one or more computing devices 504 can include one or more server computers.

The protein purification conditions system 502 can include one or more processors, such as processor 506. The one or more processors 506 can include at least one hardware processor, such as a microprocessor. In some cases, the one or more processors 506 can include a central processing unit (CPU), a graphics processing unit (GPU), or both a CPU and GPU, or other processing units. Additionally, the one or more processors 506 can include a local memory that may store program modules, program data, and/or one or more operating systems.

In addition, the protein purification conditions system 502 can include one or more computer-readable storage media, such as computer-readable storage media 508. The computer-readable storage media 508 can include volatile and nonvolatile memory and/or removable and non-removable media implemented in any type of technology for storage of information, such as computer-readable instructions, data structures, program modules, or other data. Such computer-readable storage media 508 can include, but is not limited to, RAM, ROM, EEPROM, flash memory or other memory technology, CD-ROM, digital versatile disks (DVD) or other optical storage, magnetic cassettes, magnetic tape, solid state storage, magnetic disk storage, RAID storage systems, storage arrays, network attached storage, storage area networks, cloud storage, removable storage media, or any other medium that can be used to store the desired information and that can be accessed by a computing device. Depending on the configuration of the protein purification conditions system 502, the computer-readable storage media 508 can be a type of tangible computer-readable storage media and can be a non-transitory storage media.

The protein purification conditions system 502 can include one or more communication interfaces 510 to communicate with other computing devices via one or more networks (not shown), such as one or more of the Internet, a cable network, a satellite network, a wide area wireless communication network, a wired local area network, a wireless local area network, or a public switched telephone network (PSTN).

The computer-readable storage media 508 can be used to store any number of functional components that are executable by the one or more processors 506. In many implementations, these functional components comprise instructions or programs that are executable by the one or more processors 506 and that, when executed, implement operational logic for performing the operations attributed to the protein purification conditions system 502. Functional components of the protein purification conditions system 502 that can be executed on the one or more processors 506 for implementing the various functions and features related to determining conditions for the purification of proteins, as described herein, include protein data collection and storage instructions 512, model generating instructions 514, purification conditions optimization instructions 516, and multi-well plate sampling instructions 518.

Additionally, the one or more computing devices 504 can include one or more input/output devices (not shown). The one or more input/output devices can include a display device, keyboard, a remote controller, a mouse, a printer, audio input/output devices, a speaker, a microphone, a camera, and so forth

The protein purification conditions system 502 can also include, or be coupled to, a data store 520 that can include, but is not limited to, RAM, ROM, EEPROM, flash memory, one or more hard disks, solid state drives, optical memory (e.g. CD, DVD), or other non-transient memory technologies. The data store 520 can maintain information that is utilized by the protein purification conditions system 502 to perform operations related to generating models that can be utilized to determine conditions for the purification of proteins. For example, the data store 520 can store training data 522 that can be analyzed to generate the models.

In the illustrative example of FIG. 5, the training data 522 can include sequence data for proteins where the amino acid sequences can be utilized to generate models to determine conditions for the purification of proteins. The sequence data can indicate amino acids included at various positions of the proteins that are analyzed to generate the protein purification conditions models. The training data 522 can also include structure data for proteins where the structure data can be used to generate models for determining conditions to purify proteins. The structure data can indicate secondary structures of the proteins, tertiary structure of the proteins, or both secondary structures and tertiary structures of the proteins. The training data 522 can also include biophysical properties data that includes values of various biophysical properties of proteins that can be used to generate models to determine purification conditions for various proteins. Additionally, the training data 522 can include purity and yield values for proteins that have been obtained with respect to a number of stationary phase materials at various combinations of pH levels and salt concentrations.

The protein data collection and storage instructions 508 can be executable by the one or more processors 506 to obtain and store data related to proteins that are evaluated for generating models to determine conditions for protein purification. In some implementations, the protein data collection and storage instructions 508 can obtain the training data 522 from a number of sources. In certain implementations, the protein data collection and storage instructions 512 can obtain sequence data, structure data, biophysical property data, purification conditions data, measures of performance for chromatographic processes, or combinations thereof from one or more websites and/or one or more databases that are repositories for protein related data. In additional implementations, the protein data collection and storage instructions 512 can produce one or more user interfaces that include one or more user interface elements to capture sequence data, structure data, biophysical property data, purification conditions data, and/or measures of performance for chromatographic processes. In further implementations, the protein data collection and storage instructions 512 can obtain sequence data, structure data, biophysical property data, purification conditions data, measures of performance for chromatographic processes, or combinations thereof from one or more data storage devices. The one or more data storage devices can include removable data storage devices, such as memory sticks, flash drives, or thumb drives. The one or more data storage devices can also include data stores coupled to the protein purification conditions system 502 via one or more networks, such as wired local area networks, wireless local area networks, wireless wide area networks, or combinations thereof.

The model generating instructions 514 can be executable by the one or more processors 506 to generate one or more models to determine conditions for the purification of proteins. The model generating instructions 514 can utilize the training data 522 to determine relationships between features of proteins and the performance of stationary phase materials of chromatographic techniques. In various implementations, the model generating instructions 514 can identify relationships between sequences of proteins, structures of proteins, measured attributes of proteins, or combinations thereof and yield and/or purity of one or more chromatographic techniques. The model generating instructions 514 can also determine relationships between purification conditions, such as pH values and salt concentrations, and performance of stationary phase materials. For example, the model generating instructions 514 can determine relationships between various combinations of salt concentrations and pH values and yield and/or purity for one or more chromatographic techniques.

The model generating instructions 514 can also determine an amount of influence that particular features have on performance of stationary phase materials. In particular implementations, the model generating instructions 514 can determine an amount of influence that certain portions of protein sequences have on yield and purity of proteins with respect to one or more stationary phase materials. In addition, the model generating instructions 514 can determine an impact that various structures of proteins have on yield and purity with respect to one or more stationary phase materials. Further, the model generating instructions 514 can determine an impact that measured attributes, such as turbidity, molecular weight, and protein stability, have on purity and/or yield in relation to various stationary phase materials. In certain implementations, the model generating instructions 514 can determine an amount of influence that purification conditions, such as pH levels and salt concentrations, have on the performance of one or more stationary phase materials. In various implementations, the influence of a particular protein feature on the yield and/or purity of a purification process can be accounted for in the models by assigning certain weights to the respective variables associated with the protein features included in the model.

After determining relationships between a number of features related to proteins and performance of stationary phase materials, the model generating instructions 514 can generate individual models for each stationary phase material. The models can determine yield and/or purity for proteins based on one or more inputs corresponding to characteristics of the proteins. In some situations, a minimum amount of information can be obtained for a protein in order to implement the model for the protein. For example, a model can utilize information related to at least a portion of a sequence of a protein (e.g., certain positions of the protein), a number of beta sheets associated with the protein, and a stability of the protein as indicated by differential scanning fluorimetry (DSF) to predict yield and purity of the protein at specified ranges of pH levels and salt concentrations. Continuing with this example, to implement this model, the protein purification conditions system 502 can obtain information related to the particular portion of the sequence of the protein that corresponds to the sequence portion associated with the model, the number of beta sheets, and the DSF data for the protein to determine the yield and the purity for the protein when purified with the stationary phase material associated with the model at the specified pH and salt concentrations. In certain implementations, the models can include polynomial models.

The purification conditions optimization instructions 516 can be executable by the one or more processors 506 to determine conditions for the purification of proteins that maximize yield and purity while minimizing cost of the purification process. The cost of the purification process can be related to a monetary value of implementing the purification process with a stationary phase material and can also include, in some scenarios, a difficulty in working with the respective stationary phase material and/or a durability of the stationary phase material. In some implementations, the purification conditions optimization instructions 516 can determine an optimized set of the conditions to purify proteins according to one or more models. The models used to determine the conditions to purify the proteins can be generated using at least portions of the training data 522.

The purification conditions optimization instructions 516 can determine one or more stationary phase materials to utilize to purify a particular protein. In some implementations, the purification conditions optimization instructions 516 can determine that a combination of stationary phase materials can be utilized to purify a protein. To illustrate, the purification conditions optimization instructions 516 can determine that a protein can be purified utilizing a first stationary phase material followed by purification using a second stationary phase material. Additionally, the purification conditions optimization instructions 516 can determine optimized conditions for the purification process. For example, the purification conditions optimization instructions 516 can determine a range of pH values and a range of salt concentrations that maximize yield and purity of the purification process using one or more stationary phase materials, while minimizing cost. In some cases, the purification conditions optimization instructions 516 can determine a single combination of a pH value and a salt concentration that maximize yield and purity of the purification process using one or more stationary phase materials, while minimizing cost.

The multi-well plate sampling instructions 518 can be executable by the one or more processors 506 to determine a sample of wells selected from a larger number of wells in a multi-well plate that provide conditions to purify proteins using a stationary phase material. In various implementations, a multi-well plate can include a number of wells that are each associated with a set of purification conditions. For example, individual wells of a multi-well plate can be associated with a stationary phase material, a pH level, and a salt concentration. The multi-well plate sampling instructions 518 can determine a subset of the individual wells that model a larger number of wells of the multi-well plate that are also associated with the stationary phase material. To illustrate, the multi-well plate sampling instructions 518 can identify 5 wells out of 24 that are associated with a particular stationary phase material that can be used to reproduce results obtained from the full 24 wells. That is, the multi-well plate sampling instructions 518 can identify 5 sets of purification conditions that can reproduce results of 24 sets of purification conditions with a minimum amount of error for a particular stationary phase material. In various implementations, the multi-well plate sampling instructions 518 can perform an iterative process to compare yield and purity results obtained from a subset of wells of a multi-well plate with yield and purity results obtained from a greater number of wells of the multi-well plate. The multi-well plate sampling instructions 518 can perform comparisons between the reference data that is obtained from a set of wells of a multi-well plate and various subsets of the wells of the multi-well plate until an amount of error between the yield and purity values for a particular subset of wells is minimized with respect to the reference data. After determining a subset of purification conditions for a particular stationary phase material that minimizes error with respect to the reference data, the same subset of purification conditions can be utilized in the purification of additional proteins for the stationary phase material.

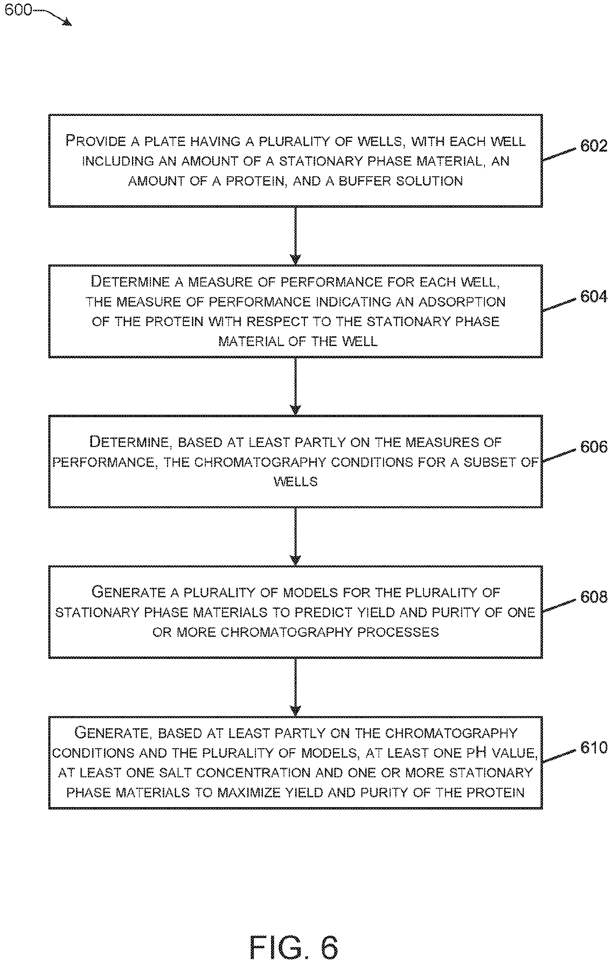

FIG. 6 illustrates an example process of determining conditions for the purification of proteins. This process (as well as each process described herein) is illustrated as logical flow graphs, each operation of which represents a sequence of operations that can, at least in part, be implemented in hardware, software, or a combination thereof. In the context of software, the operations represent computer-executable instructions stored on one or more computer-readable storage media that, when executed by one or more processors, perform the recited operations. Generally, computer-executable instructions include routines, programs, objects, components, data structures, and the like that perform particular functions or implement particular abstract data types. The order in which the operations are described is not intended to be construed as a limitation, and any number of the described operations can be combined in any order and/or in parallel to implement the process.

FIG. 6 is a flow diagram of an example process 600 to determine purification conditions for proteins using one or more chromatographic processes. At 602, the process 600 includes providing a plate having a plurality of wells with each well including (i) a stationary phase material of a plurality of stationary phase materials for column chromatography systems, (ii) an amount of a protein, and (iii) an amount of a solution having a pH and a concentration of a salt. Each well of the plurality of wells can have a different combination of the stationary phase material, pH, and concentration of the salt.

At 604, the process 600 can include determining a measure of performance for each well of the plurality of wells. The measure of performance of an individual well of the plurality of wells can indicate adsorption of the protein with respect to the stationary phase material included in the individual well. In particular implementations, the measure of performance can indicate a yield and/or a purity of a chromatographic technique that utilizes the stationary phase material included in the well.

At 606, the process 600 can include determining, based at least partly on individual measures of performance for the plurality of wells, chromatography conditions for a subset of the wells. The chromatography conditions can include a pH value and a salt concentration for each well of the subset of wells. The subset of wells can be determined by iteratively selecting various subsets of wells and identifying the subset of wells that minimizes error with respect to a reference data set.

At 608, the process 600 can include generating a plurality of models for the plurality of stationary phase materials to predict yield and purity of one or more chromatography processes. The plurality of models can be generated based at least partly on at least one of amino acid sequences of proteins, one or more structures of the proteins, or characterization data for the proteins. The characterization data can include values obtained from analytical instruments that indicate properties of the proteins.

At 610, the process 600 can include generating, based at least partly on the chromatography conditions and the plurality of models, at least one pH value of the range of pH values, at least one salt concentration of the range of salt concentrations, and one or more stationary phase materials of the plurality of stationary phase materials to maximize yield and purity of the protein.

### EXPERIMENTAL EXAMPLES

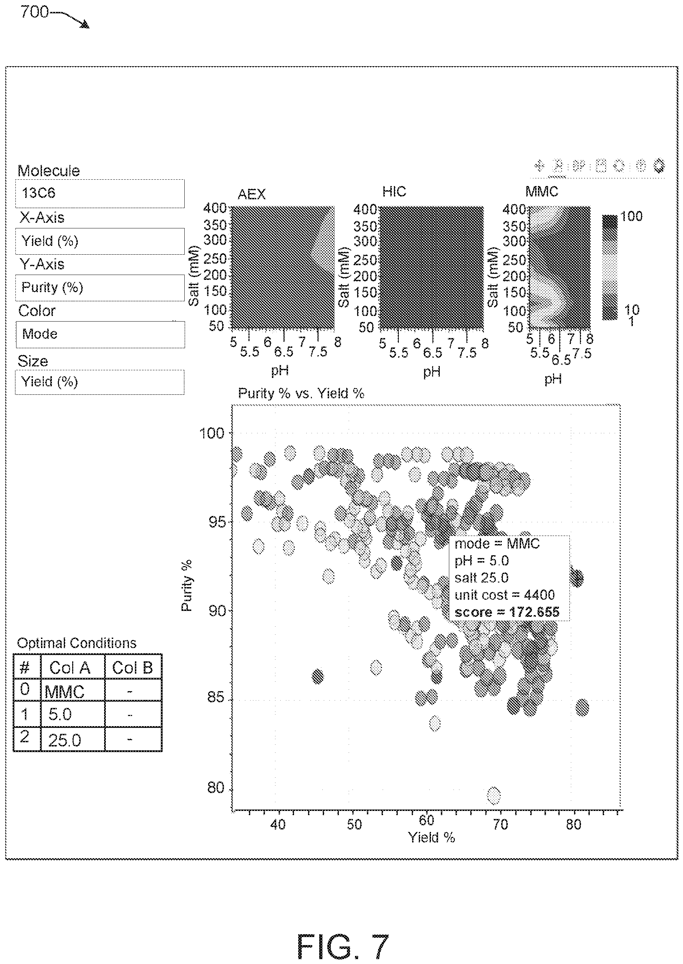

FIG. 7 illustrates a number of user interfaces that indicate yield and purity for a protein with respect to a number of pH levels and salt concentrations. Optimal conditions for purification of the protein are indicated.

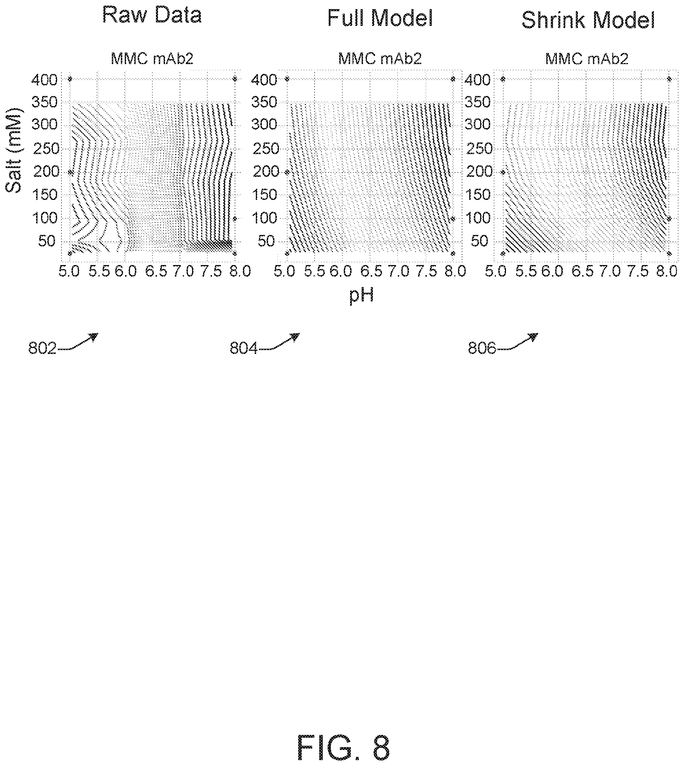

FIG. 8 illustrates results from different models that can be used to determine yield and purity for proteins using multi-mode chromatography at various pH levels and salt concentrations. 802 indicates a model based on data from each well of a set of wells included in a multi-well plate. 804 indicates a model based on data from a first subset of wells selected from the same set of wells used to produce the model indicated by 802. The model indicated at 804 is based on about 80% of the data utilized to produce the model indicated by 802. 806 indicates a model derived from data of a second subset of wells selected from the same set of wells used to produce the model indicated by 802. The model indicated at 806 is based on about 20% of the data utilized to produce the model indicated by 802.

### Introduction

High throughput screening (HTS) and miniaturization are well established strategies for streamlining downstream process development. During routine development, utilization of HTS technologies across the entire downstream process can enhance overall process understanding and better avoid process design gaps. During early phase development, HTS methods can facilitate development of multiple molecules in parallel and inform efficient, phase-appropriate work streams. The present work describes a method for integrated downstream process development in a single 96-well filter plate experiment. The plate design allows for data collection on the colloidal, chemical, and thermal stability of a product over the full range of downstream solution conditions, including low pH viral inactivation. Additionally, in the same plate, batch binding studies were performed to investigate protein-adsorbent interactions over 5 types of chromatographic media.

### Materials and Methods

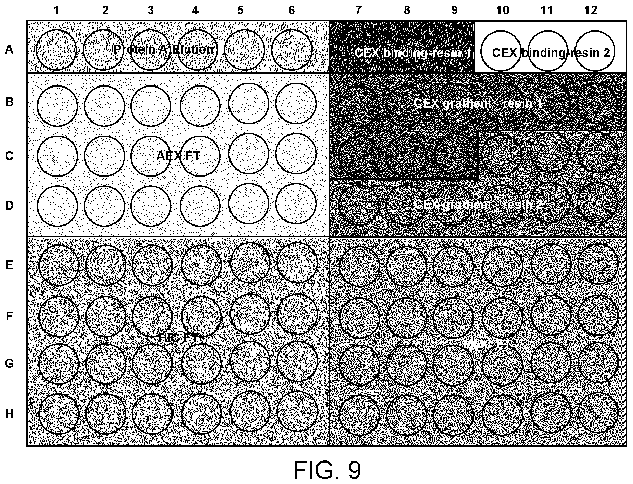

The method was implemented using a 96-well filter plate with 50 µL resin in each well. The entire method, including load and buffer preparation, was automated on a robotic liquid handling system. After the solution load preparation, the plate was loaded and incubated for 60 minutes, followed by recovery of the unbound material. Next, the resin in the wells was incubated with either a strip solution or elution buffer, depending on the mode of chromatography, followed by collection of the stripped or eluted material. The load, unbound, elution, and strip samples were analyzed for protein concentration and target impurities (e.g. HMW, HCP, or clips). Resins evaluated in the 96-well plate include protein A affinity and cation exchange (CEX) chromatographies in bind/elute mode and anion exchange (AEX), hydrophobic interaction (HIC), and mixed-mode (MMC) chromatographies in flowthrough (FT) mode. Figure 9 presents the plate configuration and Table 1 shows the operational conditions tested.
**Figure 9** **shows a 96-well filter plate configuration.** The colors indicate areas of the plate dedicated to specific resins (Protein A affinity: red, AEX: yellow, HIC: green, MMC: purple, CEX resin 1: light blue and dark grey, and CEX resin 2: dark blue and light grey).

**Table 1. Operational conditions and outputs for single 96-well filter plate experiment**

| **Resin** | **pH** | **Salt (mM)** | **Mode** | **Loading (g/Lᵣ)** | **Wells** | **Output** |
|---|---|---|---|---|---|---|
| Protein A | 3.3-3.8 | 0 | BE¹ | 20 | 6 | Recovery, HMW, low pH stability |
| CEX 1 | 5 | 0-100 | Binding | ≥ 80 | 3 | Static capacity |
| | 5 | 0-650³ | BE | 20 | 9 | Recovery, purity |
| CEX 2 | 5 | 0-100 | Binding | ≥ 80 | 3 | Static capacity |
| | 5 | 0-500³ | BE | 20 | 9 | Recovery Purity |
| AEX | 6-8 | 33-200 | FT² | 5 | 18 | Recovery, purity, contaminant removal, Kp, solution stability (load) |
| HIC | 5-8 | 25-400 | FT | 5 | 24 | |
| MMC | 5-8 | 25-400 | FT | 5 | 24 | |

| | | | | | | |
|---|---|---|---|---|---|---|
| ¹ BE = Bind and Elute ² FT = Flow Through ³ Binding at 0mM salt followed by stepwise elution to 650mM and 500mM salt over 9 wells for CEX resin 1 and CEX resin 2, respectively | | | | | | |

### Solution Stability

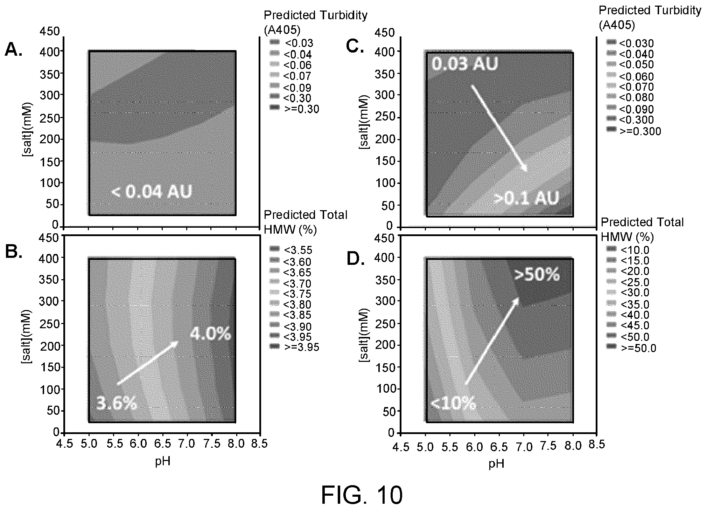

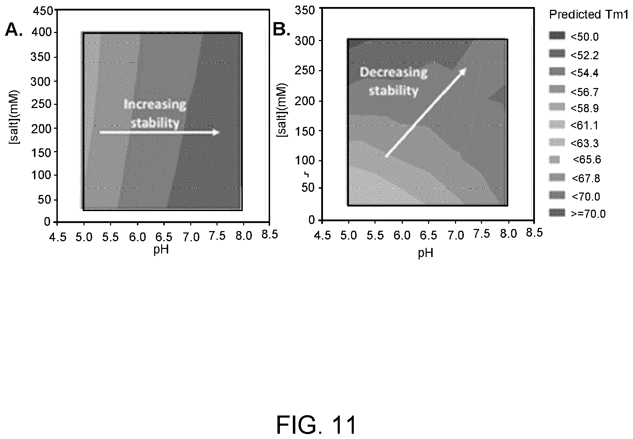

Significant insight into potential operating conditions can be gained by evaluating the solution stability of molecules. Colloidal, chemical, and thermal stability across the potential solution conditions in the downstream process was used to identify the operating space where the product is most stable and to identify conditions that minimize yield loss and aggregation. Figure 10 compares the colloidal stability by light scattering and the chemical stability by size exclusion chromatography (SEC) for 2 different monoclonal antibodies (mAbs). As shown in this example, mAb 1 is relatively stable with respect to precipitation and aggregation across the range of downstream operating conditions evaluated. Alternatively, mAb 2 precipitates at high pH and low ionic strength and displays significant aggregation at high pH and high salt strength, indicating that these solution conditions should be avoided during downstream process design. Figure 11 highlights the difference in thermal stability by differential scanning fluorimetry (DSF) for an IgG1 mAb and a Fc-fusion protein. As shown in this example, mAb 3 exhibits typical thermal stability for an IgG1 along with greater thermal stability as the pH increases. The Fc-fusion protein, in addition to an overall lower Tm, exhibits a decrease in thermal stability with an increase in pH and salt strength.
**Figure 10** **shows a comparison of solution stability for 2 mAbs for the range of downstream process conditions evaluated.** Panels A and B present turbidity and HMW, respectively, for mAb 1. Panels C and D present turbidity and HMW for mAb 2.
**Figure 11** **illustrates thermal stability by DSF for the range of downstream process conditions evaluated.** Panel A: mAb 3; panel B: Fc-fusion protein 1.

### Batch Binding

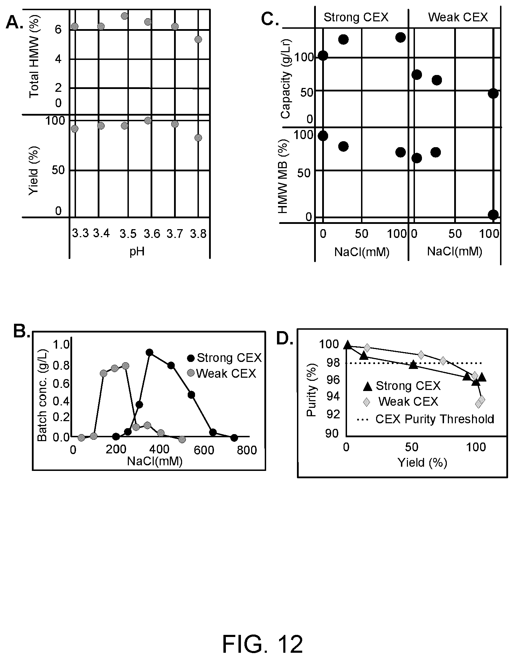

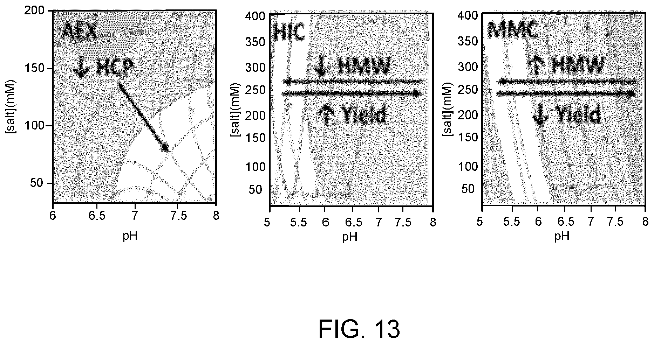

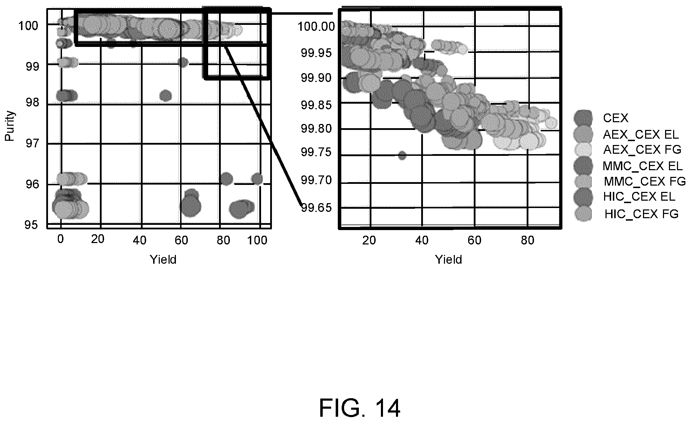

The 96-well plate was used to perform batch binding studies to investigate protein-adsorbent interactions over 5 types of chromatographic media across a wide range of pH and salt conditions. This format was used to screen protein A elution conditions; evaluate static resin capacity, gradient elution strength, and selectivity for 2 cation exchange resins; and evaluate selectivity of product and contaminants for three different resins operated in flow-through mode for a single molecule. Figures 12 and 13 present an example of the data obtained in the single plate experiment. These data can be used to estimate Protein A recovery and molecule sensitivity to low-pH viral inactivation; select a CEX resin based on elution strength, recovery and selectivity for contaminants; and, finally, identify the optimal flowthrough resin and conditions for operation. The integrated plate-based method presented herein provides insight into setpoints for individual unit operations as well as sensitivity to operating ranges. In addition, mode pairing may be employed to select resins and operating conditions across the entire process (Fig. 14)
**Figure 12** **illustrates examples of data generated during a single batch binding experiment.** Panel A: Protein A recovery vs. elution pH and pool HMW following a 60 minute low-pH hold. Panel B: Pseudo-chromatograms for 2 CEX resins. Panel C: Static capacity and HMW mass balance for 2 CEX resins. Panel D: Purity (HMW) plot for 2 CEX resins. **Figure 13** illustrates recovery and contaminant contour plots for AEX, HIC, and MMC resins operated in flowthrough mode.
**Figure 14** **illustrates purity versus yield for paired modes across entire downstream process.** Each data point represents the purity by SEC (% monomer) and recovery for different process options. The purple points are Protein A paired with CEX. The other colors indicate protein A and CEX paired with the different polish options (AEX, HIC, or MMC). panel: results for the full range of process options. Right panel: zoomed view of the red callout box, which indicates conditions that produce >70% overall recovery and > 98% purity.

The presented approach method enables an ultra-rapid development cycle wherein process development scientists determine molecule manufacturability and process operating ranges for an entire downstream process in a single plate-based experiment. The resources required for this method are approximately 100 mg of protein and 1 day of run time. In contrast, to generate comparable data using traditional bench scale chromatography systems and scale-down size columns could require 5-8 weeks of run time and in excess of 50g of protein, depending on assumptions.

When combined with miniaturized, automated robo-column chromatography to verify operating conditions, the presented method integrates into a phase-appropriate development strategy that can dramatically increase the efficiency of the downstream development cycle without sacrificing process understanding and robustness.

The subject matter described above is provided by way of illustration only and should not be construed as limiting. Furthermore, the claimed subject matter is not limited to implementations that solve any or all disadvantages noted in any part of this disclosure. Various modifications and changes can be made to the subject matter described herein without following the example configurations and applications illustrated and described, and without departing from the true scope of the present invention, which is set forth in the following claims.

## Claims

1. A method comprising:
generating yield data and purity data for a number of proteins (102, 302), the yield data and the purity data indicating yield and purity for each of a plurality of proteins (102, 302) over a range of pH values, over a range of salt concentrations, and for a stationary phase material of a column chromatography technique;
obtaining sequence data (108) indicating at least a portion of amino acid sequences of individual proteins (102, 302) of the plurality of proteins;
obtaining structure data (110) indicating one or more structures exhibited by individual proteins (102, 302) of the plurality of proteins;
generating characterization data (304, 404) for the plurality of proteins (102,302), the characterization data (304, 404) including values obtained from analytical tests that indicate values of properties of the plurality of proteins (102, 302);
generating a model (406) to predict, at one or more pH values and one or more salt concentration values (414), yield and purity of an additional protein (234) for the stationary phase material, wherein the model (406) includes a sequence component that indicates a similarity between the amino acid sequences of the plurality of proteins (102, 302) and an additional amino acid sequence of the additional protein (234) and a characterization component that indicates similarities between values of the properties for the plurality of proteins (102, 302) and additional values of the properties for the additional protein (234).

2. The method of claim 1, wherein the yield data and the purity data are determined from running a number of chromatography columns for a number of different proteins using the stationary phase material over the range of pH values and the range of salt concentrations.

3. The method of claim 1 or claim 2, wherein the yield data and the purity data are determined by analyzing solutions obtained from wells of a multi-well plate (202), individual wells of the multi-well plate (202) including an amount of a solution including the protein (102, 302) and having a pH level and a salt concentration and the multi-well plate (202) including an amount of the stationary phase material.

4. The method of any one of claims 1-3, wherein the one or more structures includes hydrophobic regions, polar regions, folds, turns, loops, or combinations thereof.

5. The method of any one of claims 1-4, wherein:
the additional protein (234) and the number of proteins (102, 302) each include a common secondary structure;
at least a portion of the amino acid sequence of the additional protein (234) and at least a portion of the amino acid sequences of the number of proteins (102, 302) have at least 75% identity;
the analytical tests produce values of one or more biophysical properties of the number of proteins (102, 302); and
a range of values for a biophysical property of the one or more biophysical properties for the additional protein (234) is within at least about 90% of a range of values for the biophysical property for the number of proteins (102, 302).

6. The method of any one of claims 1-5, wherein the characterization data (304, 404) is related to at least one of differential scanning fluorimetry measurements for the number of proteins (102, 302), molecular weight of the number of proteins (102, 302), turbidity of solutions including the number of proteins (102, 302), or combinations thereof.

7. The method of any one of claims 1-6, wherein generating a model (406) to predict, at one or more pH values and one or more salt concentration values, yield and purity of an additional protein (234) for the stationary phase material, comprises:
determining a relationship between a structure of the one or more structures exhibited by individual proteins (102, 302) of the plurality of proteins and at least one of the yield and the purity for each of the plurality of proteins (102, 302) over the range of pH values, over the range of salt concentrations, and for the stationary phase material of the column chromatography technique; and
wherein the model (406) includes at least one variable indicating a relationship between the structure and the yield and the purity for the stationary phase material.

8. A system (502) comprising:
one or more processors (506); and
one or more non-transitory computer-readable storage media (508) storing computer-readable instructions that, when executed by the one or more processors (506), perform operations comprising:
generating yield data and purity data for a number of proteins (102, 302), the yield data and the purity data indicating yield and purity for each of a plurality of proteins over a range of pH values, over a range of salt concentrations, and for a stationary phase material of a column chromatography technique;
obtaining sequence data (108) indicating at least a portion of amino acid sequences of individual proteins (102, 302) of the plurality of proteins;
obtaining structure data (110) indicating one or more structures exhibited by individual proteins (102, 302) of the plurality of proteins;
generating characterization data (304) for the plurality of proteins, the characterization data (304) including values obtained from analytical tests that indicate values of properties of the plurality of proteins; and
generating a model (406) to predict, at one or more pH values and one or more salt concentration values, yield and purity of an additional protein (234) for the stationary phase material, wherein the model (406) includes a sequence component that indicates a similarity between the amino acid sequences of the plurality of proteins and an additional amino acid sequence of the additional protein (234) and a characterization component that indicates similarities between values of the properties for the plurality of proteins and additional values of the properties for the additional protein (234).

9. The system (502) of claim 8, wherein:
the model (406) is one of a plurality of models (120, 122, 124) to predict the yield and purity for each of the plurality of proteins (102, 302) over the range of pH values and over the range of salt concentrations;
each model (120, 122, 124) of the plurality of models is associated with an individual stationary phase material of a plurality of stationary phase materials; and
each model (120, 122, 124) of the plurality of models is associated with a different stationary phase material of the plurality of stationary phase materials.

10. The system (502) of claim 9, wherein the one or more non-transitory computer-readable storage media (508) store additional computer-readable instructions that, when executed by the one or more processors (506), perform additional operations comprising:
generating, using the plurality of models (120, 122, 124) purification conditions including at least one pH value of the range of pH values, at least one salt concentration of the range of salt concentrations, and one or more stationary phase materials of the plurality of stationary phase materials for the additional protein (234).

11. The system (502) of claim 10, wherein the one or more non-transitory computer-readable storage media (508) store additional computer-readable instructions that, when executed by the one or more processors (506), perform additional operations comprising:
determining the one or more stationary phase materials based at least partly on a durability of individual stationary phase materials of the plurality of stationary phase materials;
determining an amount of the individual stationary phase materials to be utilized in a chromatography column to purify the protein; and
determining a size of the chromatography column.

12. The system (502) of claim 11, wherein the purification conditions indicate that the protein is to be purified utilizing a first stationary phase material at a first pH value and a first salt concentration followed by purification of the protein utilizing a second stationary phase material, different from the first stationary phase material, at a second pH value that is different from the first pH value and a second salt concentration that is different from the first salt concentration.

13. The system (502) of any one of claims 8-12, wherein the one or more non-transitory computer-readable storage media (508) store additional computer-readable instructions that, when executed by the one or more processors (506), perform additional operations comprising:
determining
a measure of performance for each well of a plurality of wells of a plate (202), the measure of performance of an individual well of the plurality of wells indicating adsorption of the protein with respect to the stationary phase material included in the individual well;
wherein individual wells of the plurality of wells include a stationary phase material of a column chromatography system, an amount of one or more proteins, and an amount of a solution having a pH and a concentration of a salt; and
wherein each well of the plurality of wells has a different combination of the stationary phase material, pH, and concentration of the salt.

14. The system (502) of claim 13, wherein the measure of performance includes a yield, a purity, a characteristic of the solution after a period of time, a property of a remaining amount of the protein included in the solution after the period of time, or combinations thereof.

15. The system (502) of claim 13, wherein the one or more non-transitory computer-readable storage media (508) store additional computer-readable instructions that, when executed by the one or more processors (506), perform additional operations comprising generating the plurality of models (120, 122, 124) based at least partly on the measure of performance for each well of the plurality of wells.

## Patentansprüche

1. Ein Verfahren, das Folgendes beinhaltet:
Erzeugen von Ausbeutedaten und Reinheitsdaten für eine Anzahl von Proteinen (102, 302), wobei die Ausbeutedaten und die Reinheitsdaten die Ausbeute und Reinheit für jedes von einer Vielzahl von Proteinen (102, 302) über einen Bereich von pH-Werten, über einen Bereich von Salzkonzentrationen und für ein Stationäre-Phase-Material einer Säulenchromatographietechnik anzeigen;
Erhalten von Sequenzdaten (108), die mindestens einen Anteil von Aminosäuresequenzen individueller Proteine (102, 302) von der Vielzahl von Proteinen anzeigen;
Erhalten von Strukturdaten (110), die eine oder mehrere Strukturen anzeigen, die von individuellen Proteinen (102, 302) von der Vielzahl von Proteinen gezeigt werden;
Erzeugen von Charakterisierungsdaten (304, 404) für die Vielzahl von Proteinen (102, 302), wobei die Charakterisierungsdaten (304, 404) Werte umfassen, die aus analytischen Prüfungen erhalten wurden, die Werte von Eigenschaften von der Vielzahl von Proteinen (102, 302) anzeigen;
Erzeugen eines Modells (406), um Ausbeute und Reinheit eines zusätzlichen Proteins (234) für das Stationäre-Phase-Material bei einem oder mehreren pH-Werten und einem oder mehreren Salzkonzentrationswerten (414) vorherzusagen, wobei das Modell (406) eine Sequenzkomponente, die eine Ähnlichkeit zwischen den Aminosäuresequenzen der Vielzahl von Proteinen (102, 302) und einer zusätzlichen Aminosäuresequenz des zusätzlichen Proteins (234) anzeigt, und eine Charakterisierungskomponente, die Ähnlichkeiten zwischen Werten der Eigenschaften für die Vielzahl von Proteinen (102, 302) und zusätzlichen Werten der Eigenschaften für das zusätzliche Protein (234) anzeigt, umfasst.

2. Verfahren gemäß Anspruch 1, wobei die Ausbeutedaten und die Reinheitsdaten aus dem Durchlaufen einer Anzahl von Chromatographiesäulen für eine Anzahl verschiedener Proteine unter Verwendung des Stationäre-Phase-Materials über den Bereich von pH-Werten und den Bereich von Salzkonzentrationen bestimmt werden.

3. Verfahren gemäß Anspruch 1 oder Anspruch 2, wobei die Ausbeutedaten und die Reinheitsdaten bestimmt werden, indem aus Wells einer Multiwellplatte (202) erhaltene Lösungen analysiert werden, wobei die individuellen Wells der Multiwellplatte (202) eine Menge einer Lösung umfassen, die das Protein (102, 302) umfasst und einen pH-Niveau und eine Salzkonzentration aufweist, und die Multiwellplatte (202) eine Menge des Stationäre-Phase-Materials umfasst.

4. Verfahren gemäß einem der Ansprüche 1-3, wobei die eine oder mehreren Strukturen hydrophobe Regionen, polare Regionen, Faltungen, Kehren, Schleifen oder Kombinationen davon umfassen.

5. Verfahren gemäß einem der Ansprüche 1-4, wobei:
das zusätzliche Protein (234) und die Anzahl von Proteinen (102, 302) jeweils eine gemeinsame Sekundärstruktur umfassen;
mindestens ein Anteil der Aminosäuresequenz des zusätzlichen Proteins (234) und mindestens ein Anteil der Aminosäuresequenzen der Anzahl von Proteinen (102, 302) eine mindestens 75%ige Identität aufweisen;
die analytischen Prüfungen Werte einer oder mehrerer biophysikalischer Eigenschaften der Anzahl von Proteinen (102, 302) produzieren; und
ein Bereich von Werten für eine biophysikalische Eigenschaft von der einen oder den mehreren biophysikalischen Eigenschaften für das zusätzliche Protein (234) innerhalb mindestens etwa 90 % eines Bereichs von Werten für die biophysikalische Eigenschaft für die Anzahl von Proteinen (102, 302) liegt.

6. Verfahren gemäß einem der Ansprüche 1-5, wobei die Charakterisierungsdaten (304, 404) in Zusammenhang mit mindestens einem von Differentialfluorimetriemessungen für die Anzahl von Proteinen (102, 302), Molekulargewicht der Anzahl von Proteinen (102, 302), Trübung von Lösungen, die die Anzahl von Proteinen (102, 302) umfassen, oder Kombinationen davon stehen.

7. Verfahren gemäß einem der Ansprüche 1-6, wobei das Erzeugen eines Modells (406), um die Ausbeute und Reinheit eines zusätzlichen Proteins (234) für das Stationäre-Phase-Material bei einem oder mehreren pH-Werten und einem oder mehreren Salzkonzentrationswerten vorherzusagen, Folgendes beinhaltet:
Bestimmen einer Beziehung zwischen einer Struktur von der einen oder den mehreren Strukturen, die von individuellen Proteinen (102, 302) von der Vielzahl von Proteinen gezeigt werden, und mindestens einem von der Ausbeute und der Reinheit für jedes von der Vielzahl von Proteinen (102, 302) über den Bereich von pH-Werten, über den Bereich von Salzkonzentrationen und für das Stationäre-Phase-Material der Säulenchromatographietechnik; und
wobei das Modell (406) mindestens eine Variable umfasst, die eine Beziehung zwischen der Struktur und der Ausbeute und der Reinheit für das Stationäre-Phase-Material anzeigt.

8. Ein System (502), das Folgendes beinhaltet:
einen oder mehrere Prozessoren (506); und
ein oder mehrere nichtflüchtige computerlesbare Speichermedien (508), die computerlesbare Anweisungen speichern, die, wenn sie von dem einen oder den mehreren Prozessoren (506) ausgeführt werden, Betriebsvorgänge durchführen, die Folgendes beinhalten:
Erzeugen von Ausbeutedaten und Reinheitsdaten für eine Anzahl von Proteinen (102, 302), wobei die Ausbeutedaten und die Reinheitsdaten Ausbeute und Reinheit für jedes von einer Vielzahl von Proteinen über einen Bereich von pH-Werten, über einen Bereich von Salzkonzentrationen und für ein Stationäre-Phase-Material einer Säulenchromatographietechnik anzeigen;
Erhalten von Sequenzdaten (108), die mindestens einen Anteil von Aminosäuresequenzen individueller Proteine (102, 302) von der Vielzahl von Proteinen anzeigen;
Erhalten von Strukturdaten (110), die eine oder mehrere Strukturen anzeigen, die von individuellen Proteinen (102, 302) von der Vielzahl von Proteinen gezeigt werden;
Erzeugen von Charakterisierungsdaten (304) für die Vielzahl von Proteinen, wobei die Charakterisierungsdaten (304) Werte umfassen, die aus analytischen Prüfungen erhalten wurden, die Werte von Eigenschaften der Vielzahl von Proteinen anzeigen; und
Erzeugen eines Modells (406), um Ausbeute und Reinheit eines zusätzlichen Proteins (234) für das Stationäre-Phase-Material bei einem oder mehreren pH-Werten und einem oder mehreren Salzkonzentrationswerten vorherzusagen, wobei das Modell (406) eine Sequenzkomponente, die eine Ähnlichkeit zwischen den Aminosäuresequenzen der Vielzahl von Proteinen und einer zusätzlichen Aminosäuresequenz des zusätzlichen Proteins (234) anzeigt, und eine Charakterisierungskomponente, die Ähnlichkeiten zwischen Werten der Eigenschaften für die Vielzahl von Proteinen und zusätzlichen Werten der Eigenschaften für das zusätzliche Protein (234) anzeigt, umfasst.

9. System (502) gemäß Anspruch 8, wobei:
das Modell (406) eines von einer Vielzahl von Modellen (120, 122, 124) ist, um die Ausbeute und Reinheit für jedes von der Vielzahl von Proteinen (102, 302) über den Bereich von pH-Werten und über den Bereich von Salzkonzentrationen vorherzusagen;
jedes Modell (120, 122, 124) von der Vielzahl von Modellen mit einem individuellen Stationäre-Phase-Material von einer Vielzahl von Stationäre-Phase-Materialien assoziiert ist; und
jedes Modell (120, 122, 124) von der Vielzahl von Modellen mit einem verschiedenen Stationäre-Phase-Material von der Vielzahl von Stationäre-Phase-Materialien assoziiert ist.

10. System (502) gemäß Anspruch 9, wobei das eine oder die mehreren nichtflüchtigen computerlesbaren Speichermedien (508) zusätzliche computerlesbare Anweisungen speichern, die, wenn sie von dem einen oder den mehreren Prozessoren (506) ausgeführt werden, zusätzliche Betriebsvorgänge durchführen, die Folgendes beinhalten:
Erzeugen, unter Verwendung der Vielzahl von Modellen (120, 122, 124), von Aufreinigungsbedingungen, die mindestens einen pH-Wert von dem Bereich von pH-Werten, mindestens eine Salzkonzentration von dem Bereich von Salzkonzentrationen und ein oder mehrere Stationäre-Phase-Materialien von der Vielzahl von Stationäre-Phase-Materialien für das zusätzliche Protein (234) umfassen.

11. System (502) gemäß Anspruch 10, wobei das eine oder die mehreren nichtflüchtigen computerlesbaren Speichermedien (508) zusätzliche computerlesbare Anweisungen speichern, die, wenn sie von dem einen oder den mehreren Prozessoren (506) ausgeführt werden, zusätzliche Betriebsvorgänge durchführen, die Folgendes beinhalten:
Bestimmen des einen oder der mehreren Stationäre-Phase-Materialien mindestens teilweise auf der Grundlage einer Beständigkeit individueller Stationäre-Phase-Materialien von der Vielzahl von Stationäre-Phase-Materialien;
Bestimmen einer Menge der individuellen Stationäre-Phase-Materialien, die in einer Chromatographiesäule eingesetzt werden soll, um das Protein zu reinigen; und Bestimmen einer Größe der Chromatographiesäule.

12. System (502) gemäß Anspruch 11, wobei die Aufreinigungsbedingungen anzeigen, dass das Protein unter Einsatz eines ersten Stationäre-Phase-Materials bei einem ersten pH-Wert und einer ersten Salzkonzentration aufgereinigt werden soll, gefolgt von Aufreinigung des Proteins unter Einsatz eines zweiten Stationäre-Phase-Materials, das von dem ersten Stationäre-Phase-Material verschieden ist, bei einem zweiten pH-Wert, der von dem ersten pH-Wert verschieden ist, und einer zweiten Salzkonzentration, die von der ersten Salzkonzentration verschieden ist.

13. System (502) gemäß einem der Ansprüche 8-12, wobei das eine oder die mehreren nichtflüchtigen computerlesbaren Speichermedien (508) zusätzliche computerlesbare Anweisungen speichern, die, wenn sie von dem einen oder den mehreren Prozessoren (506) ausgeführt werden, zusätzliche Betriebsvorgänge durchführen, die Folgendes beinhalten:
Bestimmen eines Leistungsmaßes für jedes Well von einer Vielzahl von Wells einer Platte (202),
wobei das Leistungsmaß eines individuellen Wells von der Vielzahl von Wells die Adsorption des Proteins im Hinblick auf das in dem individuellen Well eingeschlossene Stationäre-Phase-Material anzeigt;
wobei individuelle Wells von der Vielzahl von Wells ein Stationäre-Phase-Material eines Säulenchromatographiesystems, eine Menge von einem oder mehreren Proteinen und
eine Menge einer Lösung, die einen pH-Wert und eine Konzentration eines Salzes aufweist, umfassen; und
wobei jedes Well von der Vielzahl von Wells eine verschiedene Kombination des Stationäre-Phase-Materials, des pH-Werts und der Salzkonzentration aufweist.

14. System (502) gemäß Anspruch 13, wobei das Leistungsmaß eine Ausbeute, eine Reinheit, ein Charakteristikum der Lösung nach einem Zeitraum, eine Eigenschaft einer verbleibenden Menge des nach dem Zeitraum in der Lösung eingeschlossenen Proteins oder Kombinationen davon umfasst.

15. System (502) gemäß Anspruch 13, wobei das eine oder die mehreren nichtflüchtigen computerlesbaren Speichermedien (508) zusätzliche computerlesbare Anweisungen speichern, die, wenn sie von dem einen oder den mehreren Prozessoren (506) ausgeführt werden, zusätzliche Betriebsvorgänge durchführen, die das Erzeugen der Vielzahl von Modellen (120, 122, 124) mindestens teilweise auf der Grundlage des Leistungsmaßes für jedes Well von der Vielzahl von Wells beinhalten.

## Revendications

1. Un procédé comprenant :
la génération de données de rendement et de données de pureté pour un certain nombre de protéines (102, 302), les données de rendement et les données de pureté indiquant le rendement et la pureté pour chaque protéine d'une pluralité de protéines (102, 302) sur une gamme de valeurs de pH, sur une gamme de concentrations en sel, et pour un matériau de phase stationnaire d'une technique de chromatographie sur colonne ;
l'obtention de données de séquence (108) indiquant au moins une portion de séquences d'acides aminés de protéines individuelles (102, 302) de la pluralité de protéines ;
l'obtention de données de structure (110) indiquant une ou plusieurs structures présentées par des protéines individuelles (102, 302) de la pluralité de protéines ;
la génération de données de caractérisation (304, 404) pour la pluralité de protéines (102, 302), les données de caractérisation (304, 404) incluant des valeurs obtenues à partir d'essais analytiques qui indiquent des valeurs de propriétés de la pluralité de protéines (102, 302) ;
la génération d'un modèle (406) afin de prédire, à une ou plusieurs valeurs de pH et une ou plusieurs valeurs de concentration en sel (414), le rendement et la pureté d'une protéine supplémentaire (234) pour le matériau de phase stationnaire, dans lequel le modèle (406) inclut une composante de séquence qui indique une similarité entre les séquences d'acides aminés de la pluralité de protéines (102, 302) et une séquence d'acides aminés supplémentaire de la protéine supplémentaire (234) et une composante de caractérisation qui indique des similarités entre des valeurs des propriétés pour la pluralité de protéines (102, 302) et des valeurs supplémentaires des propriétés pour la protéine supplémentaire (234).

2. Le procédé de la revendication 1, dans lequel les données de rendement et les données de pureté sont déterminées à partir du passage dans un certain nombre de colonnes de chromatographie pour un certain nombre de protéines différentes à l'aide du matériau de phase stationnaire sur la gamme de valeurs de pH et la gamme de concentrations en sel.

3. Le procédé de la revendication 1 ou de la revendication 2, dans lequel les données de rendement et les données de pureté sont déterminées par analyse de solutions obtenues à partir de puits d'une plaque à puits multiples (202), des puits individuels de la plaque à puits multiples (202) incluant une quantité d'une solution incluant la protéine (102, 302) et ayant un niveau de pH et une concentration en sel et la plaque à puits multiples (202) incluant une quantité du matériau de phase stationnaire.

4. Le procédé de l'une quelconque des revendications 1 à 3, dans lequel les une ou plusieurs structures incluent des régions hydrophobes, des régions polaires, des plis, des coudes, des boucles, ou des combinaisons de ceux-ci.

5. Le procédé de l'une quelconque des revendications 1 à 4, dans lequel :
la protéine supplémentaire (234) et les protéines dudit nombre de protéines (102, 302) incluent chacune une structure secondaire commune ;
au moins une portion de la séquence d'acides aminés de la protéine supplémentaire (234) et au moins une portion des séquences d'acides aminés des protéines dudit nombre de protéines (102, 302) ont au moins 75 % d'identité ;
les essais analytiques produisent des valeurs d'une ou de plusieurs propriétés biophysiques des protéines dudit nombre de protéines (102, 302) ; et
une gamme de valeurs pour une propriété biophysique des une ou plusieurs propriétés biophysiques pour la protéine supplémentaire (234) se situe dans les limites d'au moins environ 90 % d'une gamme de valeurs pour la propriété biophysique pour les protéines dudit nombre de protéines (102, 302).

6. Le procédé de l'une quelconque des revendications 1 à 5, dans lequel les données de caractérisation (304, 404) concernent au moins un élément parmi des mesures de fluorimétrie différentielle à balayage pour ledit nombre de protéines (102, 302), la masse moléculaire des protéines dudit nombre de protéines (102, 302), la turbidité de solutions incluant ledit nombre de protéines (102, 302), ou des combinaisons de celles-ci.

7. Le procédé de l'une quelconque des revendications 1 à 6, dans lequel la génération d'un modèle (406) afin de prédire, à une ou plusieurs valeurs de pH et une ou plusieurs valeurs de concentration en sel, le rendement et la pureté d'une protéine supplémentaire (234) pour le matériau de phase stationnaire, comprend :
la détermination d'une relation entre une structure des une ou plusieurs structures présentées par des protéines individuelles (102, 302) de la pluralité de protéines et au moins une grandeur parmi le rendement et la pureté pour chaque protéine de la pluralité de protéines (102, 302) sur la gamme de valeurs de pH, sur la gamme de concentrations en sel, et pour le matériau de phase stationnaire de la technique de chromatographie sur colonne ; et
dans lequel le modèle (406) inclut au moins une variable indiquant une relation entre la structure et le rendement et la pureté pour le matériau de phase stationnaire.

8. Un système (502) comprenant :
un ou plusieurs processeurs (506) ; et
un ou plusieurs supports de stockage non transitoires lisibles par ordinateur (508) stockant des instructions lisibles par ordinateur qui, lorsqu'elles sont exécutées par les un ou plusieurs processeurs (506), réalisent des opérations comprenant :
la génération de données de rendement et de données de pureté pour un certain nombre de protéines (102, 302), les données de rendement et les données de pureté indiquant le rendement et la pureté pour chaque protéine d'une pluralité de protéines sur une gamme de valeurs de pH, sur une gamme de concentrations en sel, et pour un matériau de phase stationnaire d'une technique de chromatographie sur colonne ;
l'obtention de données de séquence (108) indiquant au moins une portion de séquences d'acides aminés de protéines individuelles (102, 302) de la pluralité de protéines ;
l'obtention de données de structure (110) indiquant une ou plusieurs structures présentées par des protéines individuelles (102, 302) de la pluralité de protéines ; la génération de données de caractérisation (304) pour la pluralité de protéines, les données de caractérisation (304) incluant des valeurs obtenues à partir d'essais analytiques qui indiquent des valeurs de propriétés de la pluralité de protéines ; et la génération d'un modèle (406) afin de prédire, à une ou plusieurs valeurs de pH et une ou plusieurs valeurs de concentration en sel, le rendement et la pureté d'une protéine supplémentaire (234) pour le matériau de phase stationnaire, dans lequel le modèle (406) inclut une composante de séquence qui indique une similarité entre les séquences d'acides aminés de la pluralité de protéines et une séquence d'acides aminés supplémentaire de la protéine supplémentaire (234) et une composante de caractérisation qui indique des similarités entre des valeurs des propriétés pour la pluralité de protéines et des valeurs supplémentaires des propriétés pour la protéine supplémentaire (234).

9. Le système (502) de la revendication 8, dans lequel :
le modèle (406) est un modèle d'une pluralité de modèles (120, 122, 124) afin de prédire le rendement et la pureté pour chaque protéine de la pluralité de protéines (102, 302) sur la gamme de valeurs de pH et sur la gamme de concentrations en sel ;
chaque modèle (120, 122, 124) de la pluralité de modèles est associé à un matériau de phase stationnaire individuel d'une pluralité de matériaux de phase stationnaire ; et
chaque modèle (120, 122, 124) de la pluralité de modèles est associé à un matériau de phase stationnaire différent de la pluralité de matériaux de phase stationnaire.

10. Le système (502) de la revendication 9, dans lequel les un ou plusieurs supports de stockage non transitoires lisibles par ordinateur (508) stockent des instructions lisibles par ordinateur supplémentaires qui, lorsqu'elles sont exécutées par les un ou plusieurs processeurs (506), réalisent des opérations supplémentaires comprenant :
la génération, à l'aide de la pluralité de modèles (120, 122, 124), de conditions de purification incluant au moins une valeur de pH de la gamme de valeurs de pH, au moins une concentration en sel de la gamme de concentrations en sel, et un ou
plusieurs matériaux de phase stationnaire de la pluralité de matériaux de phase stationnaire pour la protéine supplémentaire (234).

11. Le système (502) de la revendication 10, dans lequel les un ou plusieurs supports de stockage non transitoires lisibles par ordinateur (508) stockent des instructions lisibles par ordinateur supplémentaires qui, lorsqu'elles sont exécutées par les un ou plusieurs processeurs (506), réalisent des opérations supplémentaires comprenant :
la détermination des un ou plusieurs matériaux de phase stationnaire sur la base au moins en partie d'une durabilité de matériaux de phase stationnaire individuels de la pluralité de matériaux de phase stationnaire ;
la détermination d'une quantité des matériaux de phase stationnaire individuels à utiliser dans une colonne de chromatographie afin de purifier la protéine ; et
la détermination d'une taille de la colonne de chromatographie.

12. Le système (502) de la revendication 11, dans lequel les conditions de purification indiquent que la protéine doit être purifiée à l'aide d'un premier matériau de phase stationnaire à une première valeur de pH et une première concentration en sel, étape suivie par une purification de la protéine à l'aide d'un deuxième matériau de phase stationnaire, différent du premier matériau de phase stationnaire, à une deuxième valeur de pH qui est différente de la première valeur de pH et une deuxième concentration en sel qui est différente de la première concentration en sel.

13. Le système (502) de l'une quelconque des revendications 8 à 12, dans lequel les un ou plusieurs supports de stockage non transitoires lisibles par ordinateur (508) stockent des instructions lisibles par ordinateur supplémentaires qui, lorsqu'elles sont exécutées par les un ou plusieurs processeurs (506), réalisent des opérations supplémentaires comprenant :
la détermination d'une mesure de performance pour chaque puits d'une pluralité de puits d'une plaque (202),
la mesure de performance d'un puits individuel de la pluralité de puits indiquant une adsorption de la protéine par rapport au matériau de phase stationnaire inclus dans le puits individuel ;
dans lequel des puits individuels de la pluralité de puits incluent un matériau de phase stationnaire d'un système de chromatographie sur colonne, une quantité d'une ou de plusieurs protéines, et une quantité d'une solution ayant un pH et une concentration d'un sel ; et
dans lequel chaque puits de la pluralité de puits a une combinaison différente du matériau de phase stationnaire, du pH, et de la concentration du sel.

14. Le système (502) de la revendication 13, dans lequel la mesure de performance inclut un rendement, une pureté, une caractéristique de la solution après une période de temps, une propriété d'une quantité restante de la protéine incluse dans la solution après la période de temps, ou des combinaisons de ceux-ci.

15. Le système (502) de la revendication 13, dans lequel les un ou plusieurs supports de stockage non transitoires lisibles par ordinateur (508) stockent des instructions lisibles par ordinateur supplémentaires qui, lorsqu'elles sont exécutées par les un ou plusieurs processeurs (506), réalisent des opérations supplémentaires comprenant la génération de la pluralité de modèles (120, 122, 124) sur la base au moins en partie de la mesure de performance pour chaque puits de la pluralité de puits.
